# EUROPEAN PATENT SPECIFICATION

(11) **EP 2 707 017 B1**
(45) Date of publication and mention of the grant of the patent: **16.09.2015**
(21) Application number: 12723132.2
(22) Date of filing: 11.05.2012
(51) Int. Cl.: A61K 38/26, A61K 31/155, A61P 3/10

(54) **LIXISENATIDE AND METFORMIN FOR TREATMENT OF DIABETES TYPE 2**
LIXISENATID UND METFORMIN ZUR BEHANDLUNG VON TYP-2-DIABETES
LIXISENATIDE ET METFORMINE POUR LE TRAITEMENT DU DIABETE DE TYPE 2

(30) Priority: 13.05.2011 EP 11166120; 10.04.2012 EP 12163637
(43) Date of publication of application: 19.03.2014
(73) Proprietor: Sanofi-Aventis Deutschland GmbH, 65929 Frankfurt am Main (DE)
(72) Inventor: RUUS, Peter, 65926 Frankfurt am Main (DE); SILVESTRE, Louise, F-75008 Paris (FR); MIOSSEC, Patrick, F-75008 Paris (FR); PINQUIER, Jean-Louis, F-75008 Paris (FR); HINCELIN-MERY, Agnes, F-75008 Paris (FR)
(74) Representative: Weickmann & Weickmann
(86) International application number: PCT/EP2012/058747
(87) International publication number: WO 2012/156298

(56) References cited:
- EP-A1- 2 324 853
- RATNER R E ET AL: "Dose-dependent effects of the once-daily GLP-1 receptor agonist lixisenatide in patients with Type 2 diabetes inadequately controlled with metformin: A randomized, double-blind, placebo-controlled trial", DIABETIC MEDICINE, JOHN WILEY & SONS, LTD, GB, vol. 27, no. 9, 1 September 2010 (2010-09-01), pages 1024-1032, XP002626157, ISSN: 0742-3071, DOI: 10.1111/J.1464-5491.2010.03020.X [retrieved on 2010-04-30]
- ROSENSTOCK J ET AL: "Dose range effects of the new once daily GLP-1 receptor agonist AVE0010 added to metformin in type 2 diabetes", DIABETOLOGIA, SPRINGER, BERLIN, DE, vol. 51, no. Suppl. 1, 1 September 2008 (2008-09-01), page S66, XP009130805, ISSN: 0012-186X
- GALLWITZ B: "Liraglutide. GLP-1 receptor agonist, Treatment of type 2 diabetes, Treatment of obesity", DRUGS OF THE FUTURE, vol. 33, no. 1, January 2008 (2008-01), pages 13-20, XP002680596, ISSN: 0377-8282

## Description

Subject of the present invention is a pharmaceutical combination for use in reduction of glucagon levels in diabetes type 2 patients, said combination comprising (a) desPro³⁸Exendin-4(1-39)-Lys₆-NH₂ or/and a pharmaceutically acceptable salt thereof, (b) metformin or/and a pharmaceutically acceptable salt thereof, and (c) a sulfonyl urea, wherein the subject to be treated has a 2 hours postprandial plasma glucose concentration of at least 14 mmol/L.

In a healthy person the release of insulin by the pancreas is strictly coupled to the concentration of blood glucose. An increased level of blood glucose, as appears after meals, is rapidly counterbalanced by a respective increase in insulin secretion. In fasting condition the plasma insulin level drops to a basal value which is sufficient to ensure the continuous supply of glucose to insulin-sensitive organs and tissues and to keep the hepatic glucose production at a low level at night.

In contrast to diabetes type 1, there is not generally a lack of insulin in diabetes type 2 but in many cases, particularly in progressive cases, the treatment with insulin is regarded as the most suitable therapy, if required in combination with orally administered anti-diabetic drugs.

An increased glucose level in the blood over several years without initial symptoms represents a significant health risk. It could clearly be shown by the large-scale DCCT study in the USA (The Diabetes Control and Complications Trial Research Group (1993) N. Engl. J. Med. 329, 977-986) that chronically increased levels of blood glucose are a main reason for the development of diabetes complications. Examples for diabetes complications are micro and macrovascular damages that possibly manifest themselves in retinopathies, nephropathies or neuropathies and lead to blindness, renal failure and the loss of extremities and are accompanied by an increased risk of cardiovascular diseases. It can thus be concluded that an improved therapy of diabetes primarily has to aim keeping blood glucose in the physiological range as closely as possible.

A particular risk exists for overweight patients suffering from diabetes type 2, e.g. patients with a body mass index (BMI) ≥ 30. In these patients the risks of diabetes overlap with the risks of overweight, leading e.g. to an increase of cardiovascular diseases compared to diabetes type 2 patients being of a normal weight. Thus, it is particularly necessary to treat diabetes in these patients while reducing the overweight.

Metformin is a biguanide hypoglycemic agent used in the treatment of non-insulin-dependent diabetes mellitus (diabetes mellitus type 2) not responding to dietary modification. Metformin improves glycemic control by improving insulin sensitivity and decreasing intestinal absorption of glucose. Metformin is usually administered orally. However, control diabetes mellitus type 2 in obese patients by metformin may be insufficient. Thus, in these patients, additional measures for controlling diabetes mellitus type 2 may be required.

The compound desPro³⁶Exendin-4(1-39)-Lys₆-NH₂ (AVE0010, lixisenatide) is a derivative of Exendin-4. AVE0010 is disclosed as SEQ ID NO:93 in WO 01/04156:

Exendins are a group of peptides which can lower blood glucose concentration. The Exendin analogue desPro36Exendin-4(1-39)-Lys₆-NH₂ is characterised by C-terminal truncation of the native Exendin-4 sequence. DesPro³⁶Exendin-4(1-39)-Lys₆-NH₂ comprises six C-terminal lysine residues not present in Exendin-4.

In the context of the present invention, desPro³⁶Exendin-4(1-39)-Lys₆-NH₂ includes pharmaceutically acceptable salts thereof. The person skilled in the art knows pharmaceutically acceptable salts of desPro³⁶Exendin-4(1-39)-Lys₆-NH₂. A preferred pharmaceutically acceptable salt of desPro³⁶Exendin-4(1-39)-Lys₆-NH₂ employed in the present invention is acetate.

In Example 1 of the present invention, it has been demonstrated in diabetes type 2 patients that Lixisenatide (AVE0010) in an add-on therapy to metformin significantly improved glycemic control:
- Lixisenatide significantly reduced the corrected plasma glucose AUC_{0:30-4:30h} (h*mg/dL) from baseline: -227.25 compared to -72.83 in the liraglutide group.
- Lixisenatide reduced the increase in plasma glucose after a standardized breakfast to a much greater extent compared to liraglutide.
- There was a substantial modification of PPG excursion at Day 28 in the lixisenatide group, with a significant effect on maximum PPG levels (mg/dL): -70.43 in the lixisenatide group compared to -24.93 in the liraglutide group with a mean treatment difference estimate of -45.50 for lixisenatide compared to liraglutide. This difference was statistically significant (p<0.0001).
- The number of patients with two-hour postmeal plasma glucose levels below 140 mg/dL after 4 weeks of treatment (Day 28) was higher in the lixisenatide group.
- The 24-hour plasma glucose profiles for lixisenatide and liraglutide treatments at Day 28 compared to Day -1 exhibited an overall reduction in plasma glucose, with decreases in the peak glucose levels that occur in response to meal ingestion.
- Mean HbA1C levels decreased in both treatment groups.
- Decreased AUC for plasma glucagon level was more pronounced in the lixisenatide group compared to the liraglutide group.

Example 2 of the present invention relates to glycemic control in diabetes type 2 patients in asian counties (China, Malaysia, Thailand, and Hong Kong). These patients are not adequately controlled with metformin and a sulfonyl urea alone. It has been found that in these patients of asian or/and oriental race, at week 24 of the study, a significant improvement of glycemic control could be achieved by the combination of lixisenatide and metformin compared with placebo (metformin alone):
- The efficacy of lixisenatide vs. placebo was demonstrated by a significant decrease in HbA_{1c} in the lixisenatide group (-0.83%) compared with the placebo group (-0.47%). The LS mean difference vs. placebo is -0.36% (Table 10 in Example 2). Table 28 demonstrated a similar effect in the sub-group of Chinese patients.
- The analysis of HbA_{1c} responders demonstrated a statistically significant treatment difference between lixisenatide and placebo groups. 32.4% of patients in the lixisenatide group achieved a HbA_{1c} ≤ 6.5%, whereas in the placebo group, only 18.1% achieved this value. 53 % of patients in the lixisenatide group achieved a HbA_{1c} <7%, vs. 38.8% in the placebo group (Table 11 in Example 2).
- For 2-hour post-prandial plasma glucose (PPG) after a standardized meal, the lixisenatide group demonstrated a statistically significant improvement over the placebo group with a LS mean difference of -4.28 mmol/L (Table 12 in Example 2). The analysis of glucose excursion showed an LS mean difference of -3.99 mmol/L in the lixisenatide group compared with placebo (Table 17 of Example 2).
- The between-group difference in fasting plasma glucose (FPG) compared to placebo was also statistically significant for the lixisenatide group with a LS mean difference of -0.48 mmol/L (Table 13 of Example 2).
- The overall safety of the combination of lixisentide and metformin was satisfactory in asian/oriental patients.

Disclosed herein is a pharmaceutical combination for use in glycemic control in diabetes type 2 patients, said combination comprising
(a) desPro³⁶Exendin-4(1-39)-Lys₆-NH₂ or/and a pharmaceutically acceptable salt thereof, and
(b) metformin or/and a pharmaceutically acceptable salt thereof.

As demonstrated by the Examples of the present invention, the combination as described herein can be used for improving glycemic control. In the present invention, "improvement of glycemic control" or "glycemic control" in particular refers to improvement of postprandial plasma glucose concentration, improvement of fasting plasma glucose concentration, or/ and improvement of the HbA_{1c} value.

An aspect of the present invention is a pharmaceutical combination for use in the reduction of the plasma glucagon level in diabetes type 2 patients, said combination comprising
(a) desPro³⁶Exendin-4(1-39)-Lys₆-NH₂ or/and a pharmaceutically acceptable salt thereof,
(b) metformin or/and a pharmaceutically acceptable salt thereof, and
(c) a sulfonyl urea,
wherein the subject to be treated has a 2 hours postprandial plasma glucose concentration of at least 14 mmol/L.

As demonstrated by Example 1 of the present invention, the combination as described herein can be used for decreasing the plasma glucagon level.

Metformin is the international nonproprietary name of 1,1-dimethylbiguanide (CAS Number 657-24-9). In the present invention, the term "metformin" includes any pharmaceutically acceptable salt thereof.

In the present invention, metformin may be administered orally. The skilled person knows formulations of metformin suitable for treatment of diabetes type 2 by oral administration. Metformin may be administered to a subject in need thereof, in an amount sufficient to induce a therapeutic effect. Metformin may be administered in a dose of at least 1.0 g/day or at least 1.5 g/day. For oral administration, metformin may be formulated in a solid dosage form, such as a tablet or pill. Metformin may be formulated with suitable pharmaceutically acceptable carriers, adjuvants, or/and auxiliary substances.

In the present invention, desPro³⁶Exendin-4(1-39)-Lys₆-NH₂ or/and a pharmaceutically acceptable salt may be administered in an add-on therapy to administration of metformin and a sulfonyl urea.

In the present invention, the terms "add-on", "add-on treatment" and "add-on therapy" relate to treatment of diabetes mellitus type 2 with metformin and desPro³⁶Exendin-4(1-39)-Lys₆-NH₂. Metformin and desPro³⁶Exendin-4(1-39)-Lys₆-NH₂ may be administered within a time interval of 24 h. Metformin and desPro³⁶Exendin-4(1-39)-Lys₆-NH₂ each may be administered in a once-a-day-dosage. Metformin and desPro³⁶Exendin-4(1-39)-Lys₆-NH₂ may be administered by different administration routes. Metformin may be administered orally, and desPro³⁶Exendin-4(1-39)-Lys₆-NH₂ may be administered parenterally.

The combination of the present invention comprises a sulfonyl urea. In the combination, the sulfonyl urea may be administered orally. The skilled person knows suitable formulations of sulfonyl ureas. The sulfonyl urea may be administered in an add-on treatment to the combination of desPro³⁶Exendin-4(1-39)-Lys₆-NH₂ and metformin, as described herein.

The sulfonyl urea can be selected from Glibenclamide, Glibenclamide MR, Gliclazide, Gliclazide LM, Glimepiride, Glipizide, Glipizide XL, Gliquidone, and Tolbutamide. In specific embodiments, any of the specific sulfonyl ureas disclosed herein can be combined with a specific aspect of the combination of desPro³⁶Exendin-4(1-39)-Lys₆-NH₂ and Metformin, as described herein.

A preferred dose of Glibenclamide is ≤ 10 mg/day, 10 - 20 mg/day, or ≥ 20 mg/day.

A preferred dose of Glibenclamide MR is ≤ 6 mg/day, 6 - 12 mg/day, or ≥ 12 mg/day.

A preferred dose of Gliclazide is ≤ 160 mg/day, 160 - 320 mg/day, or ≥ 320 mg/day.

A preferred dose of Gliclazide LM is ≤ 60 mg/day, 60 - 120 mg/day, or ≥ 120 mg/day.

A preferred dose of Glimepiride is ≤ 4 mg/day, 4 - 8 mg/day, or ≥ 8 mg/day.

A preferred dose of Glipizide is ≤ 20 mg/day, 20 - 40 mg/day, or ≥ 40 mg/day.

A preferred dose of Glipizide XL is ≤ 10 mg/day, 10 - 20 mg/day, or ≥ 20 mg/day.

A preferred dose of Gliquidone is ≤ 60 mg/day, 60 - 90 mg/day, or ≥ 90 mg/day.

A preferred dose of Tolbutamide is ≤ 1500 mg/day, or ≥ 1500 mg/day.

The subject to be treated by the combinaton of the present invention can be a subject of asian or/and oriental race. In Example 2 of the present invention, it has been found that in patients of asian or/and oriental race a significant improvement of glycemic control could be achieved by the combination of lixisenatide, metformin and a sulfonyl urea compared with placebo (metformin and a sulfonyl urea alone).

The subject to be treated by the medicament or combination of the present invention may be a subject suffering from diabetes type 2.

The subject to be treated by the medicament or combination of the present invention suffering from diabetes type 2 may be a subject suffering from diabetes type 2, wherein diabetes type 2 is not adequately controlled by treatment with metformin alone, for instance with a dose of at least 1.0 g/day metformin or at least 1.5 g/day metformin for 3 months. In the present invention, a subject the diabetes type 2 of which is not adequately controlled may have a HbA1c value in the range of 7 % to 10%.

The subject to be treated by the medicament or combination of the present invention suffering from diabetes type 2 may be an obese subject. In the present invention, an obese subject may have a body mass index of at least 30 kg/m².

The subject to be treated by the medicament or combination of the present invention suffering from diabetes type 2 may have a normal body weight. In the present invention, a subject having normal body weight may have a body mass index in the range of 17 kg/m² to 25 kg/m², or 17 kg/m² to <30 kg/m².

The subject to be treated by the medicament or combination of the present invention may be an adult subject. The subject may have an age of at least 18 years of may have an age in the range of 18 to 80 years, of 18 to 50 years, or 40 to 80 years, or 50 to 60 years. The subject may be younger than 50 years.

The subject to be treated by the medicament or combination of the present invention preferably does not receive an antidiabetic treatment, for instance by insulin or/and related compounds.

The subject to be treated by the medicament or combination of the present invention may suffer from diabetes mellitus type 2 for at least 1 year or at least 2 years. In particular, in the subject to be treated, diabetes mellitus type 2 has been diagnosed at least 1 year or at least 2 years before onset of therapy by the medicament or combination of the present invention.

The subject to be treated may have a HbA_{1c} value of at least about 8 % or at least about 7,5%. The subject may also have a HbA_{1c} value of about 7 to about 10 %. The examples of the present invention demonstrate that treatment by desPro³⁶Exendin-4(1-39)-Lys₆-NH₂ results in a reduction of the HbA_{1c} value in diabetes type 2 patients.

In yet another aspect of the present invention, the combination as described herein can be used for improving the HbA_{1c} value in a patient suffering from diabetes type 2. Improving the HbA_{1c} value means that the HbA_{1c} value is reduced below 6.5% or 7%, for example after treatment for at least one month, at least two months, or at least three months.

Also described herein is that the combination as described herein can be used for improving glucose tolerance in a patient suffering from diabetes type 2. Improving glucose tolerance means that the postprandial plasma glucose concentration is reduced by the active agent of the present invention. Reduction means in particular that the plasma glucose concentration reaches normoglycemic values or at least approaches these values.

In the present invention, normoglycemic values are blood glucose concentrations of in particular 60 - 140 mg/dl (corresponding to 3,3 bis 7,8 mM/L). This range refers in particular to blood glucose concentrations under fasting conditions and postprandial conditions.

The subject to be treated has a 2 hours postprandial plasma glucose concentration of at least 14 mmol/L. This plasma glucose concentration exceeds normoglycemic concentrations.

The subject to be treated may have a glucose excursion of at least 2 mmol/L, at least 3 mmol/L, at least 4 mmol/L or at least 5 mmol/L. In the present invention, the glucose excursion is in particular the difference of the 2 hours postprandial plasma glucose concentration and the plasma glucose concentration 30 minutes prior to a meal test.

"Postprandial" is a term that is well known to a person skilled in the art of diabetology. The term "postprandial" describes in particular the phase after a meal or/and exposure to glucose under experimental conditions. In a healthy person this phase is characterised by an increase and subsequent decrease in blood glucose concentration. The term "postprandial" or "postprandial phase" typically ends up to 2h after a meal or/and exposure to glucose.

The subject to be treated as disclosed herein may have a fasting plasma glucose concentration of at least 8 mmol/L, at least 8,5 mmol/L or at least 9 mmol/L. These plasma glucose concentrations exceed normoglycemic concentrations.

It is described herein that the combination as described herein can be used for improving (i.e. reducing) fasting plasma glucose in a patient suffering from diabetes type 2. Reduction means in particular that the plasma glucose concentration reaches normoglycemic values or at least approaches these values.

The combination of the present invention can be used in the treatment of one or more of the medical indications described herein, for example in treatment of diabetes type 2 patients, or for conditions associated with diabetes type 2, such as improvement of glycemic control, reduction of the fasting plasma glucose concentration, for the improvement of glucose excursion, reduction of the postprandial plasma glucose concentration, improvement of glucose tolerance, improving the HbA_{1c} value, reduction of plasma glucagon level, weight loss or/and prevention of weight gain.

In the present invention, desPro³⁶Exendin-4(1-39)-Lys₆-NH₂ or/and the pharmaceutically acceptable salt thereof may be administered to a subject in need thereof, in an amount sufficient to induce a therapeutic effect.

In the present invention, desPro³⁶Exendin-4(1-39)-Lys₆-NH₂ or/and the pharmaceutically acceptable salt thereof may be formulated with suitable pharmaceutically acceptable carriers, adjuvants, or/and auxiliary substances.

The compound desPro³⁶Exendin-4(1-39)-Lys₆-NH₂ or/and a pharmaceutically acceptable salt thereof may be administered parenterally, e.g. by injection (such as by intramuscular or by subcutaneous injection). Suitable injection devices, for instance the so-called "pens" comprising a cartridge comprising the active ingredient, and an injection needle, are known. The compound desPro³⁶Exendin-4(1-39)-Lys₆-NH₂ or/and a pharmaceutically acceptable salt thereof may be administered in a suitable amount, for instance in an amount in the range of 10 to 15 µg per dose or 15 to 20 µg per dose.

In the present invention, desPro³⁶Exendin-4(1-39)-Lys₆-NH₂ or/and a pharmaceutically acceptable salt thereof may be administered in a daily dose in the range of 10 to 20 µg, in the range of 10 to 15 µg, or in the range of 15 to 20 µg. DesPro³⁶Exendin-4(1-39)-Lys₆-NH₂ or/and a pharmaceutically acceptable salt thereof may be administered by one injection per day.

In the present invention, desPro³⁶Exendin-4(1-39)-Lys₆-NH₂ or/and a pharmaceutically acceptable salt thereof may be provided in a liquid composition. The skilled person knows liquid compositions of desPro³⁶Exendin-4(1-39)-Lys₆-NH₂ suitable for parenteral administration. A liquid composition of the present invention may have an acidic or a physiologic pH. An acidic pH preferably is in the range of pH 1 - 6.8, pH 3.5 - 6.8, or pH 3.5 - 5. A physiologic pH preferably is in the range of pH 2.5 - 8.5, pH 4.0 - 8.5, or pH 6.0 - 8.5. The pH may be adjusted by a pharmaceutically acceptable diluted acid (typically HCl) or pharmaceutically acceptable diluted base (typically NaOH).

The liquid composition comprising desPro³⁶Exendin-4(1-39)-Lys₆-NH₂ or/and a pharmaceutically acceptable salt thereof may comprise a suitable preservative. A suitable preservative may be selected from phenol, m-cresol, benzyl alcohol and p-hydroxybenzoic acid ester. A preferred preservative is m-cresol.

The liquid composition comprising desPro³⁶Exendin-4(1-39)-Lys₆-NH₂ or/and a pharmaceutically acceptable salt thereof may comprise a tonicity agent. A suitable tonicity agent may be selected from glycerol, lactose, sorbitol, mannitol, glucose, NaCl, calcium or magnesium containing compounds such as CaCl₂. The concentration of glycerol, lactose, sorbitol, mannitol and glucose may be in the range of 100 - 250 mM. The concentration of NaCl may be up to 150 mM. A preferred tonicity agent is glycerol.

The liquid composition comprising desPro³⁶Exendin-4(1-39)-Lys₆-NH₂ or/and a pharmaceutically acceptable salt thereof may comprise methionine from 0.5 µg/mL to 20 µg/mL, preferably from 1 µg /ml to 5 µg/ml. Preferably, the liquid composition comprises L-methionine.

Also described herein is a method for improvement of glycemic control in diabetes type 2 patients, said method comprising administering desPro³⁶Exendin-4(1-39)-Lys₆-NH₂ or/and a pharmaceutically acceptable salt thereof, in combination with metformin to a subject in need thereof. In particular, the combination as described herein may be administered. In the method of the present invention, the subject may be the subject defined herein.

Also described herein is a method for reducing the plasma glucagon level in diabetes type 2 patients, said method comprising administering desPro³⁶Exendin-4(1-39)-Lys₆-NH₂ or/and a pharmaceutically acceptable salt thereof, in combination with metformin to a subject in need thereof. In particular, the combination as described herein may be administered. In the method of the present invention, the subject may be the subject defined herein.

Also described herein is the use of the combination as described herein for the manufacture of a medicament for the treatment of a medical indication, as described herein. For example, the combination of the present invention can be used for the manufacture of a medicament for the treatment of diabetes type 2 patients, or for the treatment of conditions associated with diabetes type 2, such as improvement of glycemic control, reduction of the fasting plasma glucose concentration, for the improvement of glucose excursion, reduction of the postprandial plasma glucose concentration, improving the HbA_{1c} value, or/and improvement of glucose tolerance. The combination of the present invention can also be used for the manufacture of a medicament for the reduction of plasma glucagon level in diabetes type 2 patients. The medicament can be formulated as described herein. For example the medicament can comprise a parenteral formulation of desPro³⁶Exendin-4(1-39)-Lys₆-NH₂ or/and a pharmaceutically acceptable salt thereof, and an oral formulation of metformin or/and a pharmaceutically acceptable salt thereof. The medicament can further comprise a formulation of a sulfonyl urea, as described herein. In particular, the formulation of the sulfonyl urea is an oral formulation.

The invention is further illustrated by the following examples and figures.

### Figure legends

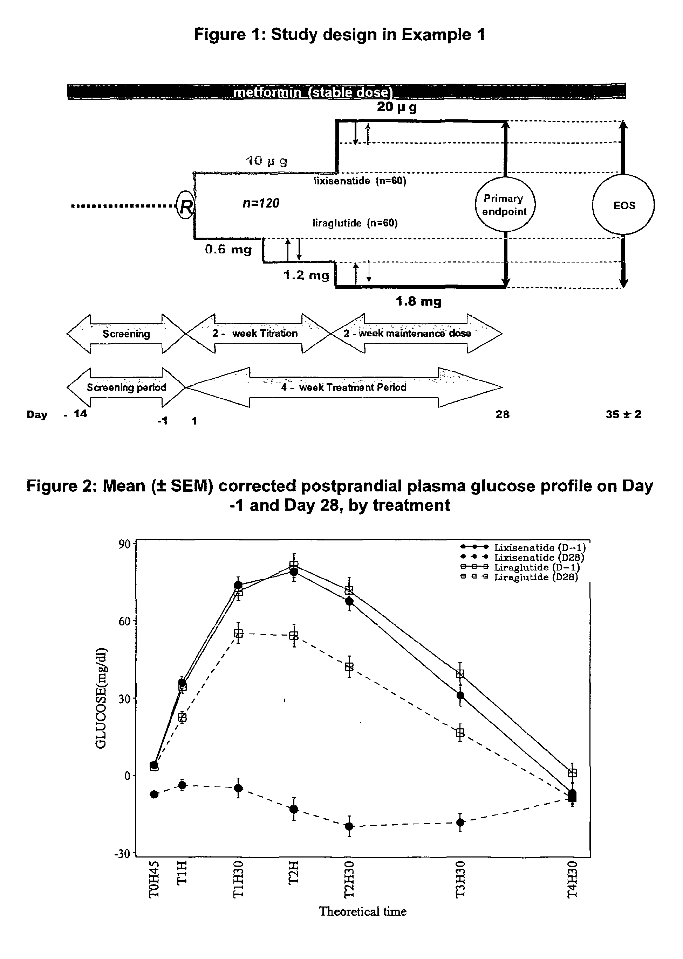

**Figure 1****:** Mean (± SEM) corrected postprandial plasma glucose profile on Day -1 and Day 28, by treatment
**Figure 2****:** Study design in Example 1.

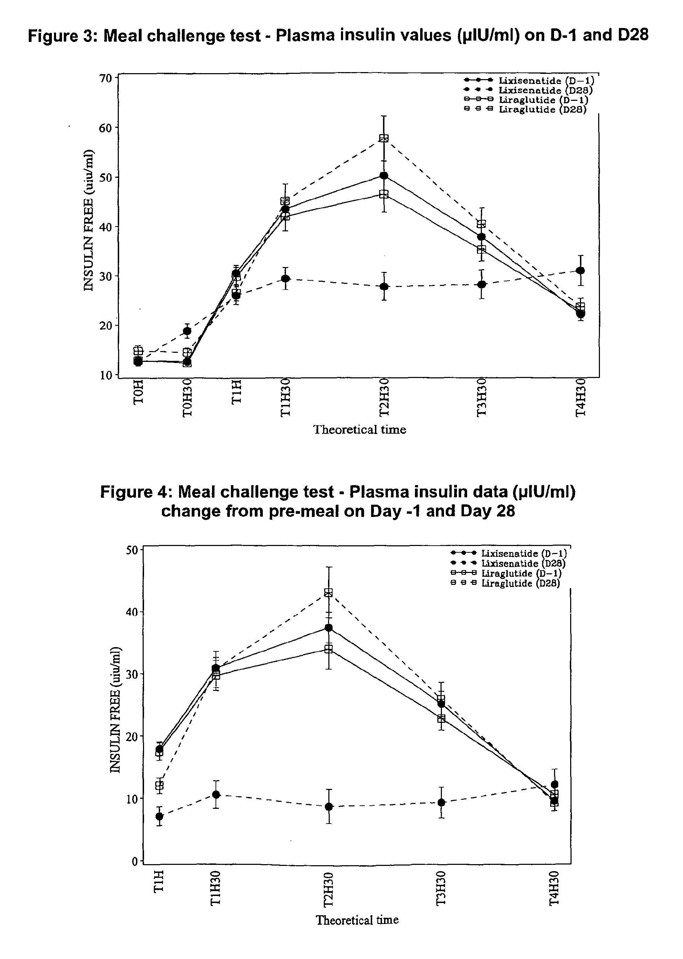

**Figure 3****:** Meal challenge test - Plasma insulin values (µlU/ml) on D-1 and D28.
**Figure 4****:** Meal challenge test - Plasma insulin data (µlU/ml) change from pre-meal on Day -1 and Day 28.

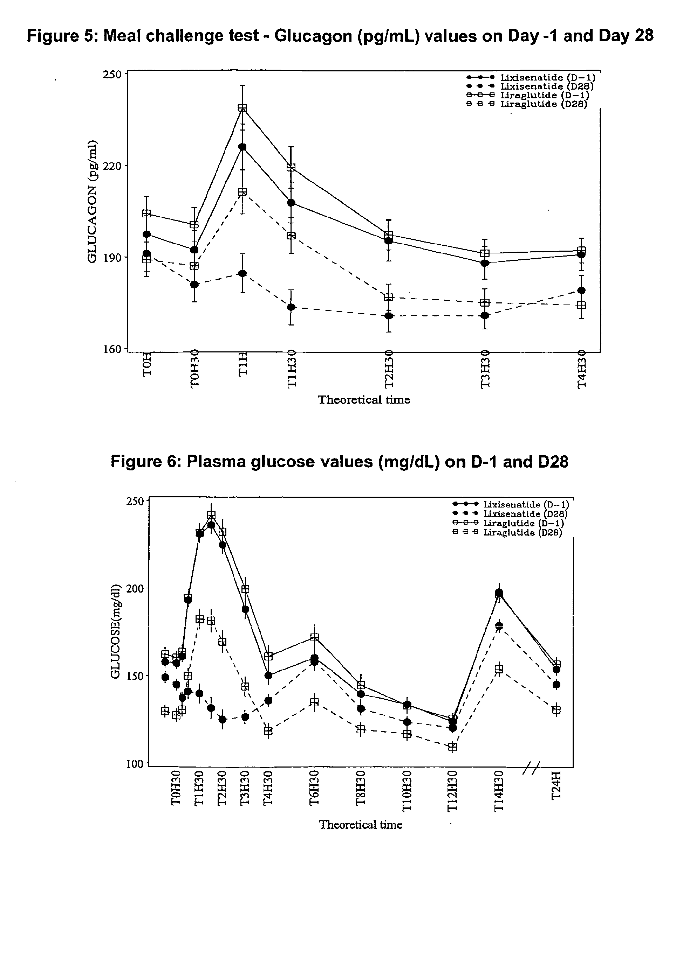

**Figure 5****:** Meal challenge test - Glucagon (pg/mL) values on Day -1 and Day 28.
**Figure 6****:** Plasma glucose values (mg/dL) on D-1 and D28.
**Figure 7****:** Study design in Example 2.
**Figure 8****:** Kaplan-Meier plot of time to treatment discontinuation due to any reason - Randomized population.
**Figure 9****:** Plot of mean change in HbA_{1c} (%) from baseline by visit and at Week 24 - mlTT population. LOCF = Last observation carried forward. Note: The analysis excluded measurements obtained after the introduction of rescue medication and/or after the treatment cessation plus 3 days.
**Figure 10****:** Plot of mean change in fasting plasma glucose (mmol/L) from baseline by visit and at Week 24 - mlTT population. LOCF = Last observation carried forward. Note: The analysis excluded measurements obtained after the introduction of rescue medication and/or after the treatment cessation plus 1 day.
**Figure 11****:** Plot of mean change in body weight (kg) from baseline by visit and at Week 24 - mITT population. LOCF = Last observation carried forward. Note: The analysis excluded measurements obtained after the introduction of rescue medication and/or after the treatment cessation plus 3 days.

### EXAMPLE 1

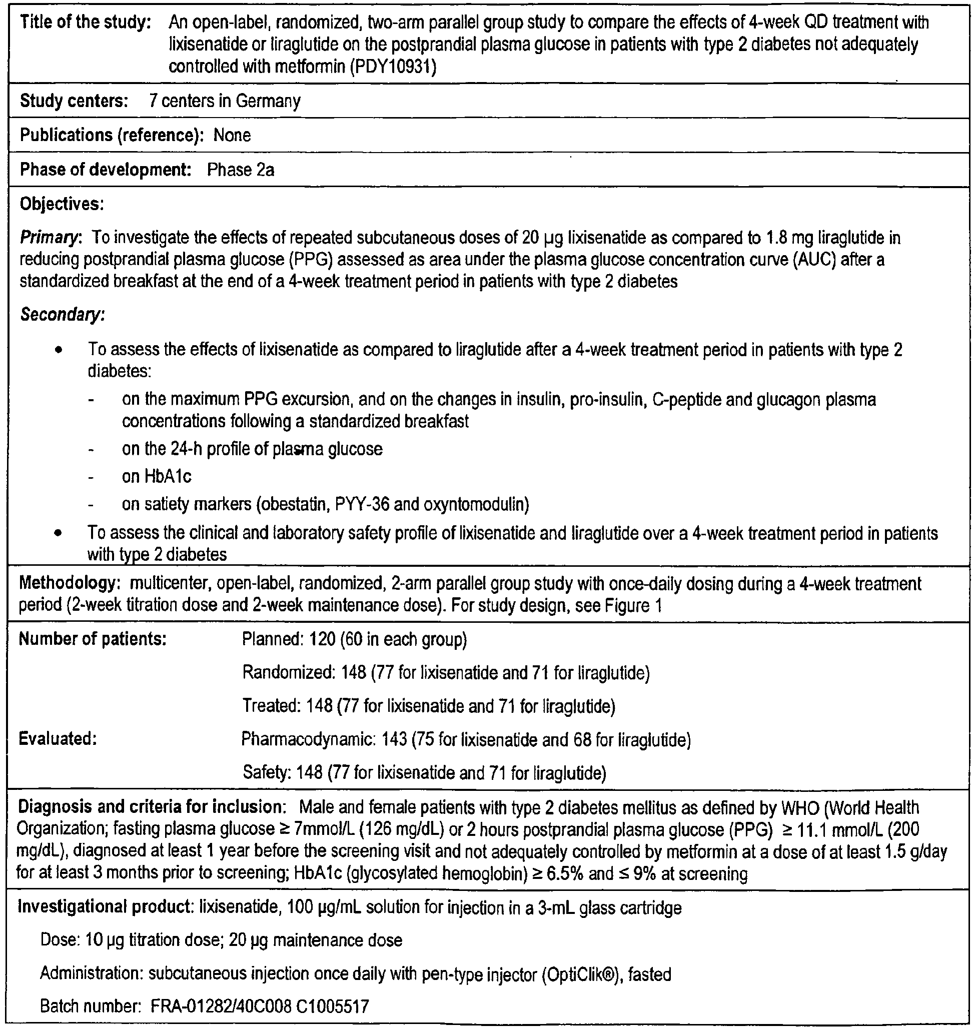

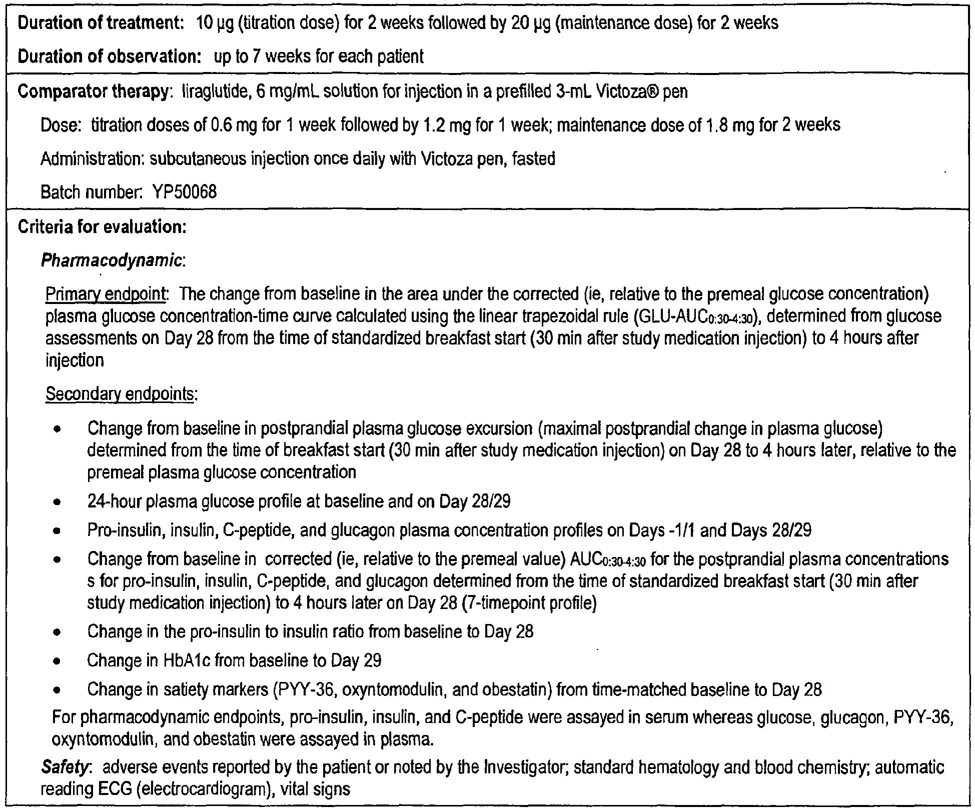

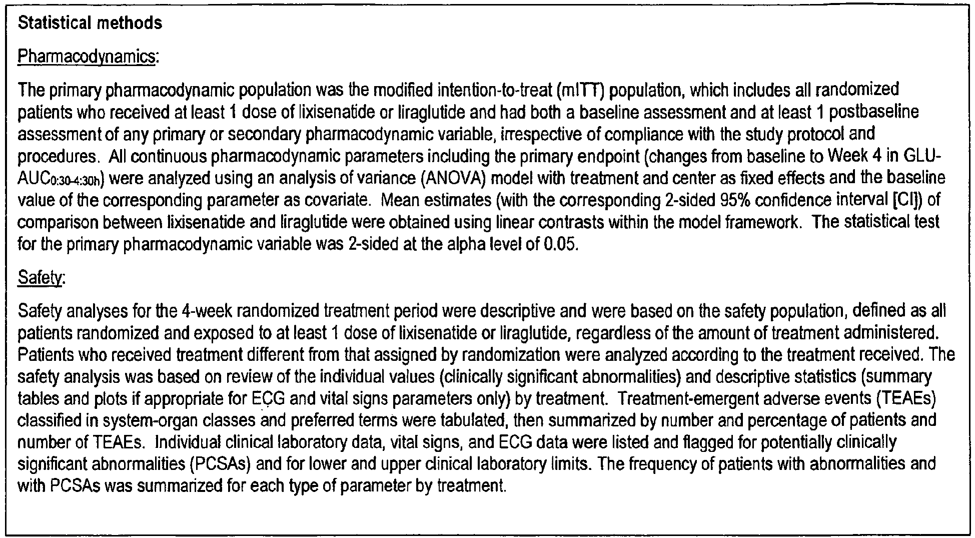

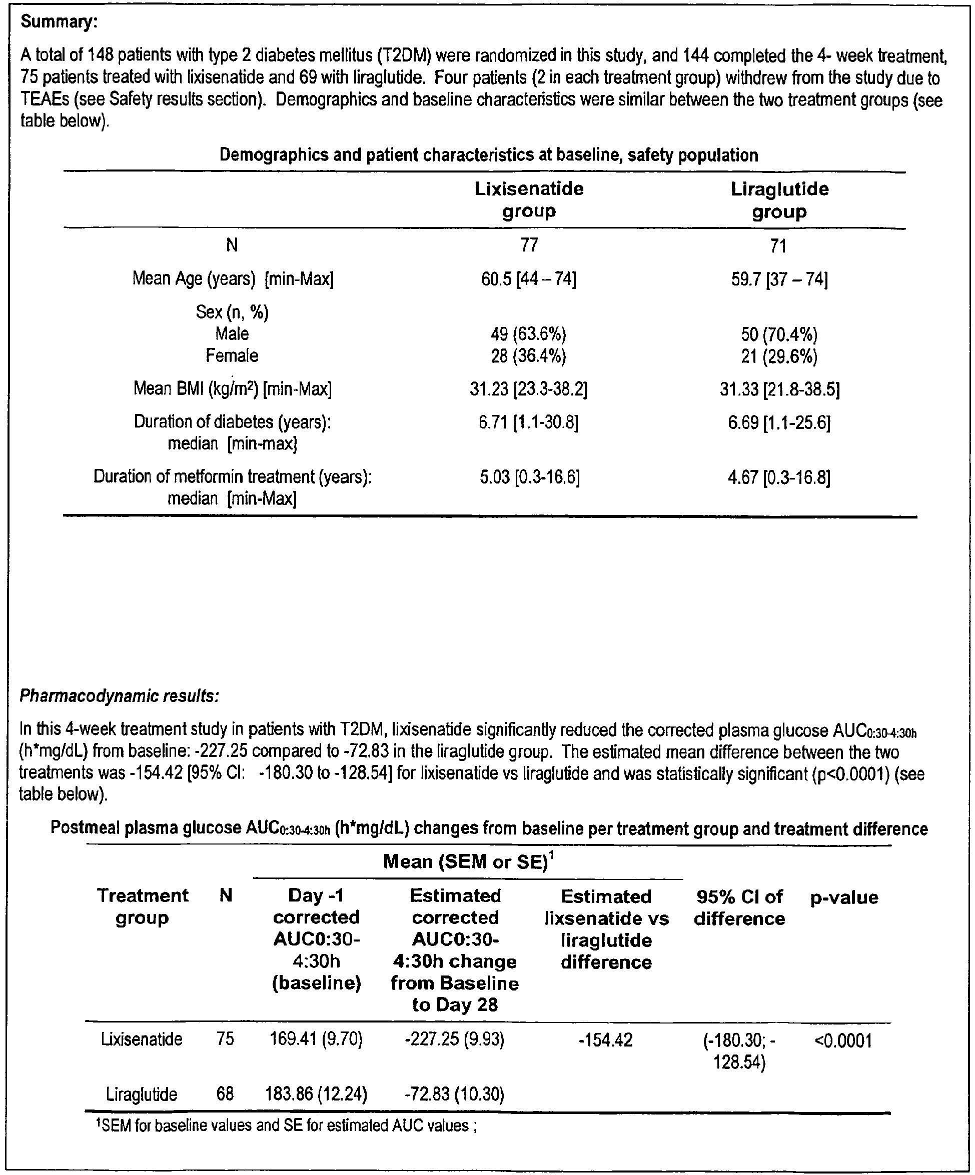

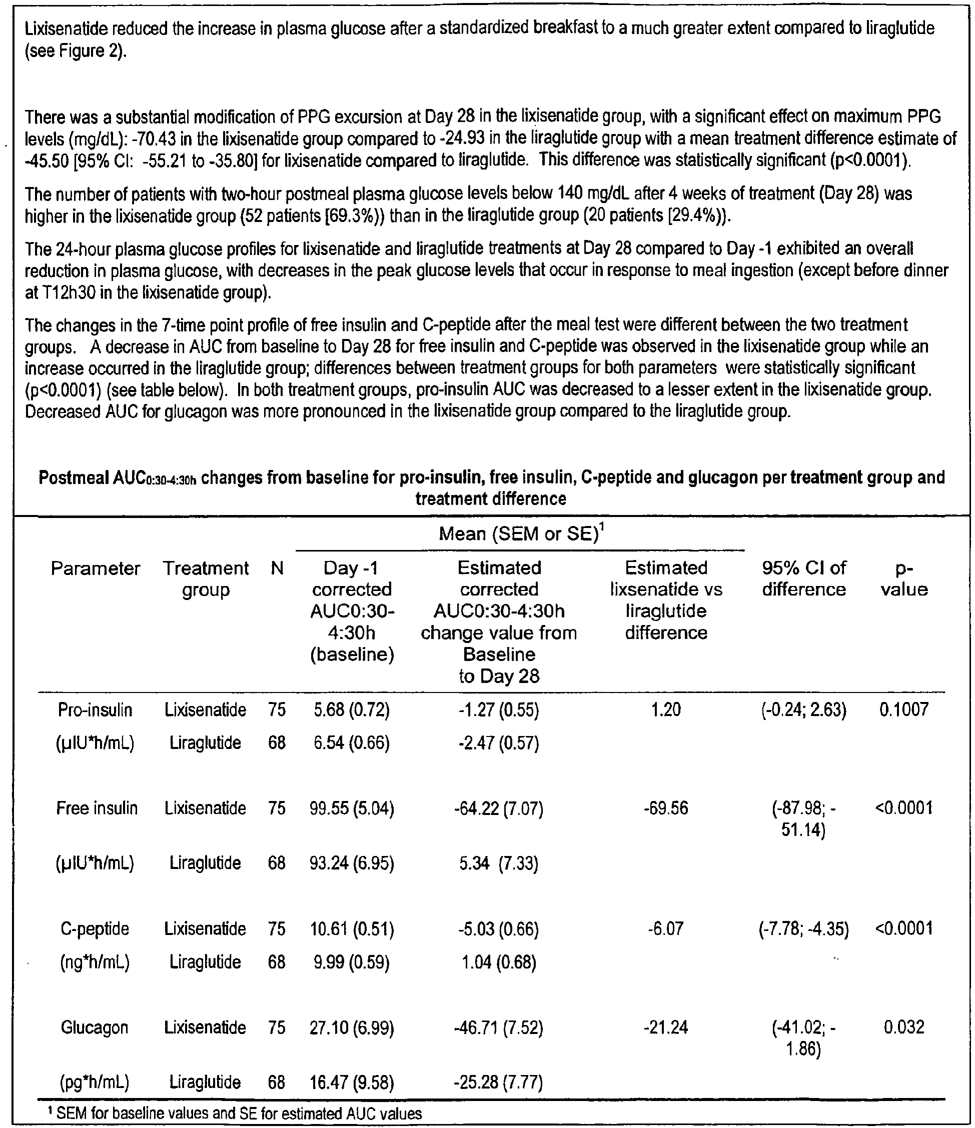

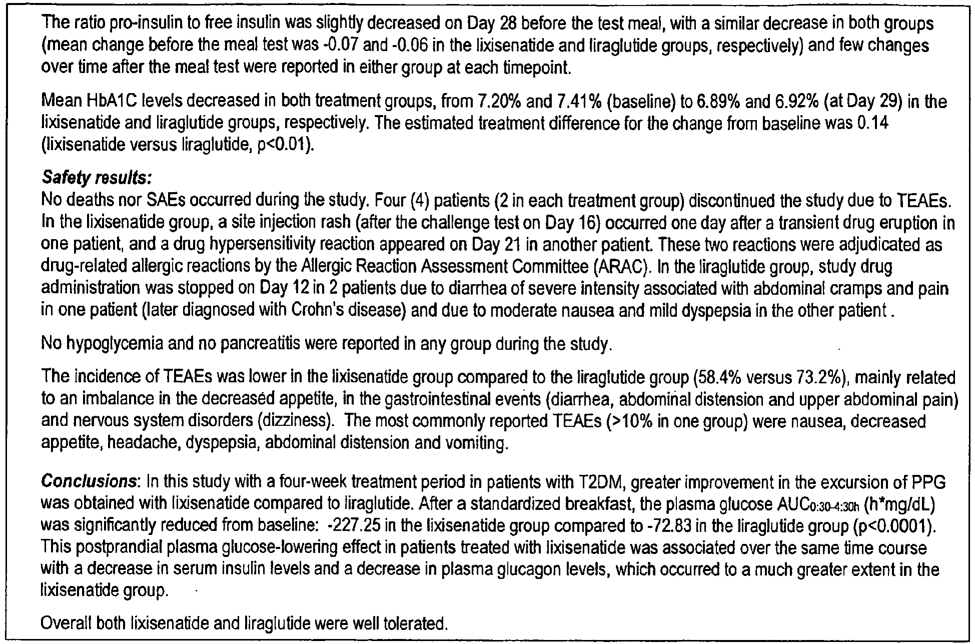

### EXAMPLE 2

### SUMMARY

This Example describes a randomized, double-blind, placebo-controlled, 2-arm parallel-group, balanced design, multinational study comparing lixisenatide treatment with placebo in type 2 diabetes mellitus (T2DM) patients insufficiently controlled by metformin with or without sulfonylurea. The study was conducted in 37 centers of 4 countries or areas (China, Malaysia, Thailand, and Hong Kong). The primary objective of this study was to assess the effects on glycemic control of lixisenatide in comparison to placebo as an add-on treatment to metformin with or without sulfonylurea in terms of HbA_{1c} reduction over a period of 24 weeks in patients with type 2 diabetes mellitus (T2DM).

A total of 391 patients were randomized to one of the two treatment groups (196 in the lixisenatide group and 195 in the placebo group). One randomized patient in placebo group was not exposed to study treatment due to personal reason. In total, 390 patients were exposed to double-blind treatment. The demographic and patient baseline characteristics were generally similar across the treatment groups. Of the 390 patients, 363 (93.1%) completed the 24-week double-blind treatment (179 patients [91.3%] in the lixisenatide group and 184 patients [94.8%] in the placebo group). Two patients (1 patient [0.5%] in each group) were excluded from the modified intent-to-treat (mITT) population for efficacy analyses due to lack of post-baseline efficacy data.

For the primary endpoint of HbA_{1c}, the efficacy of lixisenatide vs. placebo was demonstrated based on the pre-specified primary analysis of the least squared (LS) mean changes from baseline to Week 24 in HbA_{1c} (-0.83% and -0.47% in the lixisenatide and placebo groups, respectively; LS mean difference vs. placebo=-0.36%; 95% confidence interval [CI]: -0.551,-0.162; p-value=0.0004).

The analysis of HbA_{1c} responders (ie, patients with HbA_{1c} ≤6.5 or <7% at Week 24) using the Cochran-Mantel Haenszel (CMH) method also showed a statistically significant treatment difference between lixisenatide and placebo groups (for HbA_{1c} ≤ 6.5%, 32.4% in the lixisenatide group vs. 18.1% in the placebo group, p-value=0.001; for HbA_{1c} <7%, 53% in the lixisenatide group vs. 38.8% in the placebo group, p-value=0.003).

For 2-hour post-prandial plasma glucose (PPG) after a standardized meal, the lixisenatide group demonstrated a statistically significant improvement over the placebo group with a LS mean difference of 4.28 mmol/L (pvalue <0.0001). The analysis of glucose excursion showed similar results. The between-group difference in fasting plasma glucose (FPG) compared to placebo was also statistically significant for the lixisenatide group with a LS mean difference of 0.48 mmol/L (pvalue=0.0109). For body weight, a similar decrease of up to 1.5 kg was observed in both treatment groups and no statistically significant difference was observed (LS mean difference lixisenatide vs. placebo=0.27 kg; pvalue=0.2960). The percentage of patients requiring rescue therapy was 3.6% in the lixisenatide group and 6.7%.in the placebo group.

The incidence of treatment emergent adverse events (TEAEs) was higher in the lixisenatide-treated group compared to the placebo-treated group (64.3% and 47.4%, respectively). No patient had on-treatment SAEs leading to death. The number of patients with serious TEAEs were similar in both treatment groups (3 [1.5%] and 4 [2.1%] in the lixisenatide and placebo groups, respectively). More patients in the lixisenatide group (11 patients [5.6%]) discontinued treatment due to TEAE than those in the placebo group (3 patients [1.5%]), mainly due to adverse events (AE) from the gastrointestinal disorders system organ class (SOC). The most commonly reported TEAE in lixisenatide group was nausea, which is consistent with the known safety profile of glucagon-like peptide-1 (GLP-1) receptor agonists. A higher percentage of patients had nausea in the lixisenatide treatment group compared with the placebo treatment group (32 patients [16.3%] and 5 patients [2.6%], respectively). These events occurred more frequently at the beginning of the study. The second most frequently reported TEAE in the lixisenatide-treated group was hypoglycemia (18 [9.2%] lixisenatide vs. 9 [4.6%] placebo). In total, 16 patients [4.1%] had symptomatic hypoglycemia events as defined in the protocol: 11 patients (5.6%) in the lixisenatide group and 5 patients (2.6%) in the placebo group. The incidence rates of symptomatic hypoglycemia with blood glucose <60 mg/dL were exactly the same in the two treatment groups (3 patients [1.5%] in each group). None of the symptomatic hypoglycemia events were serious or severe in intensity. A higher percentage of patients had dizziness and vomiting in the lixisenatide group compared with the placebo group (for dizziness, 17 patients [8.7%] and 8 patients [4.1%], respectively; for vomiting, 15 patients [7.7%] and 2 patients [1.0%], respectively). A total of 3 patients (2 patients [1.0%] on lixisenatide and 1 patient [0.5%] on placebo) had TEAEs adjudicated as allergic reactions by the Allergic Reaction Assessment Committee (ARAC), out of which 2 events from 2 patients (anaphylactic shock and injection site reaction) in lixisenatide group were considered to be possibly related to the investigational product (IP). No patient with suspected pancreatitis or increase of calcitonin was reported in either treatment group.

In summary, the results of the study demonstrated the superior efficacy of treatment with lixisenatide compared with placebo in terms of glycemic control as evidenced by the change in HbA_{1c}, 2-hour PPG, and FPG reduction from baseline to Week 24 and HbA_{1c} responder rates at Week 24. Lixisenatide was well tolerated during the 24-week treatment period. Incidences of serious TEAEs were similar in the lixisenatide and placebo groups. A higher percentage of patients treated with lixisenatide compared with placebo experienced symptomatic hypoglycemia. However, the incidence rates of symptomatic hypoglycemia with blood glucose <60 mg/dL were exactly the same in the two treatment groups. Nausea, dizziness and vomiting were reported more frequently with lixisenatide than with placebo. No unexpected specific safety concern was observed during the trial. Overall, lixisenatide was well tolerated and effective when compared to placebo therapy in T2DM patients insufficiently controlled by metformin with or without sulfonylurea.

### 1 OBJECTIVES

### 1.1 PRIMARY OBJECTIVE

The primary objective of this study was to assess the effects on glycemic control of lixisenatide in comparison to placebo as an add-on treatment to metformin with or without sulfonylurea in terms of HbA_{1c} reduction over a period of 24 weeks in patients with T2DM.

### 1.2 SECONDARY OBJECTIVE(S)

The secondary objectives of this study were:
- To assess the effects of lixisenatide over 24 weeks on:
   - Percentage of patients reaching HbA_{1c} <7% or HbA_{1c} ≤6.5%,
   - FPG,
   - Two-hour PPG and glucose excursion during standardized meal test (approximately 50% of all randomized patients),
   - Body weight,
- To assess the safety and tolerability of lixisenatide
- To assess lixisenatide pharmacokinetic (PK) and anti-lixisenatide antibody development

### 2 TRIAL DESIGN

The study was a double-blind, randomized, placebo-controlled, 2-arm parallel-group, balanced design (1:1 ratio), multinational study with 190 patients planned in each arm. The study was double-blind with regard to active and placebo treatments. The study drug volume (ie, dose of active drug or matching placebo during titration and maintenance phases) was not blinded.

The patients were stratified by HbA_{1c} (< 8%, ≥ 8%) and sulfonylurea use (Yes, No) at screening. The number of patients in each of the sulfonylurea stratum (with sulfonylurea, without sulfonylurea) was planned to be balanced.

The study comprised 3 periods: 1) an up-to 3-week screening period, which included an up-to 2-week initial screening phase and a 1-week single-blind placebo run-in phase; 2) a 24-week double-blind, placebo-controlled treatment period; 3) a 3-day safety follow-up period for all the patients after permanent IP discontinuation (except for patients who prematurely discontinue the study treatment).

One-step dose increase regimen was used in the trial. During the double-blind period, the starting dose per injection was 10 µg lixisenatide or volume matched placebo administered once-a-day (QD) within 1 hour (ie, from 0 to 60 minutes) before breakfast. The dose per injection was increased after 2 weeks to 20 µg, provided safety and tolerability did not prevent dose increase up to the target treatment level of 20 µg/injection or volume matched placebo from Visit 5 (week 2) onwards throughout the entire study for all the patients.

For backgroud therapy, the metformin dose was kept stable at its baseline dose of at least 1.0 g/day and no more than 1.5 g/day throughout the study. In patients with sulfonylurea given on top of metformin, the dose of sulfonylurea was decreased by 25% to 50% at randomization in order to decrease the risk of hypoglycemia in patients with a screening HbA_{1c}<8%; in patients with HbA_{1c} ≥8% at screening the dose of sulfonylurea was kept stable at its baseline dose of at least the maximal effective dose (ie, half of the maximum recommended dose according to local labeling).

Patients who prematurely discontinued the study treatment continued the study up to the scheduled date of study completion. They were followed-up according to the study procedures as specified in the protocol (except 3-day safety post-treatment follow-up, meal test and PK assessments).

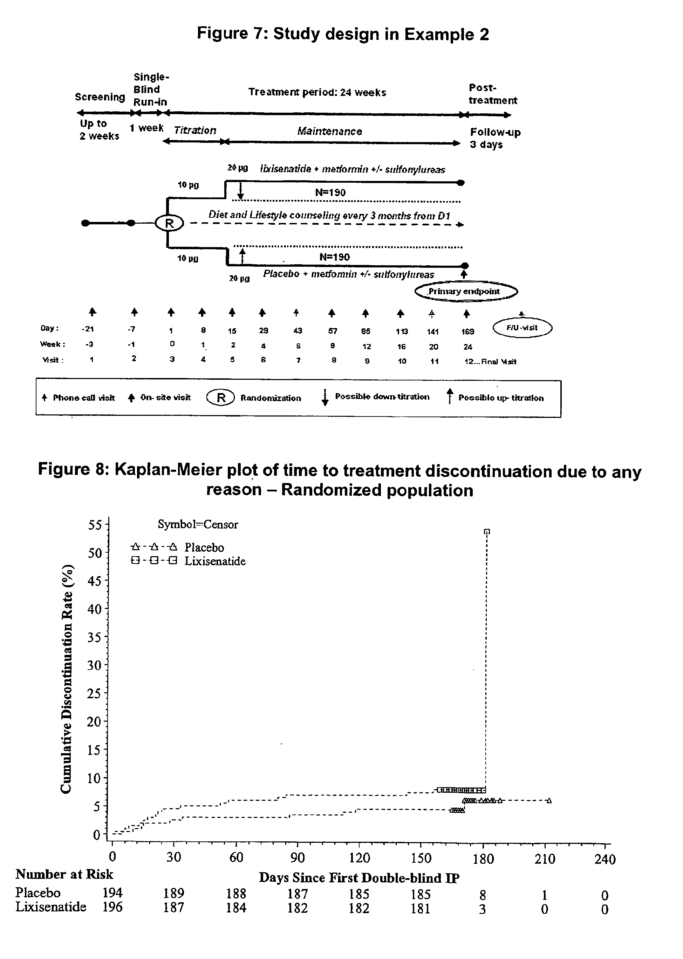

The study duration per patients was 27 weeks ± 10 days (up to 2 weeks screening + 1 week run-in + 24 weeks double-blind treatment + 3 days follow-up). For details, see Figure 7.

### 3 PRIMARY AND KEY SECONDARY ENDPOINTS

### 3.1 PRIMARY ENDPOINT

The primary efficacy variable was the absolute change in HbA_{1c} from baseline to Week 24, which was defined as: HbA_{1c} value at Week 24-HbA_{1c} value at baseline.

If a patient permanently discontinued the treatment or received rescue therapy during the 24-week double-blind treatment period or did not have HbA_{1c} value at week 24, the last post-baseline HbA_{1c} measurement during the 24-week double-blind on-treatment period was used as HbA_{1c} value at week 24 or in case of rescue the last value before rescue (Last Observation Carry Forward [LOCF] procedure).

### 3.2 SECONDARY ENDPOINTS

### 3.2.1 Efficacy endpoints

For secondary efficacy variables, the same procedure for handling missing assessments/early discontinuation was applied as for the primary efficacy variable.

### Continuous variables

- Change in FPG (mmol/L) from baseline to Week 24
- Change in 2-hour PPG (mmol/L) after a standardized meal from baseline to Week 24
- Change in glucose excursion (2hour PPG-plasma glucose 30 minutes prior to the meal test before study drug administration) (mmol/L) after a standardized meal challenge test from baseline to Week 24
- Change in body weight (kg) from baseline to Week 24

### Categorical variables

- Percentage of patients with HbA_{1c}<7% at Week 24
- Percentage of patients with HbA_{1c} ≤6.5% at Week 24
- Percentage of patients requiring rescue therapy during the 24week double-blind treatment period
- Percentage of patients with ≥5% weight loss (kg) from baseline to Week 24

### 3.2.2 Safety endpoints

The safety analysis was based on the reported TEAEs and other safety information including symptomatic hypoglycemia and severe symptomatic hypoglycemia, local tolerability at injection site, allergic events (as adjudicated by ARAC), suspected pancreatitis, increased calcitonin, vital signs, 12-lead electrocardiogram (ECG) and safety laboratory tests.

Major cardiovascular events were also collected and adjudicated by a Cardiovascular Adjudication Committee (CAC). The adjudicated and confirmed events by CAC from this study and other lixisenatide Phase 3 studies will be pooled for analyses and summarized in a separate report based on the statistical analysis plan for the overall cardiovascular assessment of lixisenatide.

### 4 SAMPLE SIZE CALCULATION ASSUMPTIONS

The sample size calculations were performed based on the primary efficacy variable of HbA_{1c} absolute change from baseline to week 24.

A total of 380 patients (190 per arm) were expected to provide a power of 96% to detect a difference of 0.5% in the absolute change in HbA_{1c} from baseline to Week 24 between lixisenatide treatment group and placebo group, assuming a common SD of 1.3% with a 2-sided test at the 5% significance level. The sample size calculations were based upon the 2-sample test and made using nQuery® Advisor 6.01.

### 5 STATISTICAL METHODS

### 5.1 ANALYSIS POPULATIONS

The mITT population consisted of all randomized patients who received at least one dose of double-blind IP, and had both a baseline assessment and at least one post-baseline assessment of efficacy variables, irrespective of compliance with the study protocol and procedures.

The safety population was defined as all randomized patients who took at least one dose of the double-blind IP.

### 5.2 PRIMARY EFFICACY ANALYSIS

The primary efficacy variable (change in HbA_{1c} from baseline to Week 24) was analyzed using an analysis of covariance (ANCOVA) model with treatment groups (lixisenatide and placebo), randomization strata of screening HbA_{1c} (<8.0, ≥8.0 %), randomization strata of screening sulfonylurea use (Yes, No) and country as fixed effects and using the baseline HbA_{1c} value as a covariate. Difference between lixisenatide and placebo and two-sided 95% CI as wells as p-value were estimated within the framework of ANCOVA.

The primary analysis of the primary efficacy variable was performed based on the mITT population and the measurements obtained during the double-blind on-treatment period for efficacy variables. The double-blind on-treatment period for efficacy variables except those from the meal challenge test was defined as the time from the first dose of the double-blind IP up to 3 days (except for FPG by central laboratory, which was up to 1 day) after the last dose of the double-blind IP injection, or up to the introduction of the rescue therapy, whichever was the earliest. The LOCF procedure was used by taking this last available post-baseline on-treatment HbA_{1c} measurement (before the initiation of the new medication in the event of rescue therapy) as the HbA_{1c} value at week 24.

### 5.3 SECONDARY EFFICACY ANALYSIS

The double-blind on-treatment period for efficacy variables from the meal challenge test including PPG and glucose excursion was defined as the time from the first dose of the double-blind IP up to the date of the last dose of the double-blind IP injection, or up to the introduction of the rescue therapy, whichever was the earliest.

Once the primary variable was statistically significant at α=0.05, the testing procedure was performed to test the following secondary efficacy variables by the following prioritized order. The tests stopped as soon as an endpoint was found not statistically significant at α=0.05.
1. Change in 2hour PPG (mmol/L) after a standardized meal test from baseline to Week 24,
2. Change in FPG (mmol/L) from baseline to Week 24,
3. Change in body weight (kg) from baseline to Week 24,
4. Percentage of patients requiring rescue therapy during the 24week double-blind treatment period.

All continuous secondary efficacy variables at week 24 as described in Section 3.2.1 were analyzed using the similar approach and ANCOVA model as described above for the primary analysis of the primary efficacy endpoint. The adjusted estimates of the treatment mean difference between lixisenatide and placebo and two-sided 95% CIs were provided.

The following categorical secondary efficacy variables at Week 24 were analyzed using a CMH method stratified on randomization strata (screening HbA_{1c} [<8.0, ≥ 8%] and sulfonylurea use at screening [Yes, No]):
- Percentage of patients with HbA_{1c}<7.0% at Week 24,
- Percentage of patients with HbA_{1c} ≤6.5% at Week 24,
- Percentage of patients requiring rescue therapy during the 24week double-blind treatment period.

Number and percentage of patients with ≥5% weight loss at Week 24 were presented by treatment groups.

### 5.4 SAFETY ANALYSIS

The safety analyses were primarily based on the on-treatment period. The on-treatment period was defined as the time from the first dose of double-blind IP up to 3 days after the last dose of IP administration regardless of rescue status. The 3-day interval was chosen based on the half-life of the lixisenatide (approximately 5 times the half-life).

The summary of safety results (descriptive statistics or frequency tables) was presented by treatment groups.

### 6 RESULTS

### 6.1 STUDY PATIENTS

### 6.1.1 Patient accountability

The study was conducted in 37 centers of 4 countries or areas (China, Malaysia, Thailand, and Hong Kong). A total of 655 patients were screened and 391 were randomized to one of the two treatment groups. The main reason for screening failure was HbA_{1c} value at the screening visit out of the defined protocol ranges (147 patients [22.4%]).

Table 1 provides the number of patients included in each analysis population. One randomized patient (in placebo group) was not exposed to the study treatment as the patient withdrew from study. The other 390 randomized patients were exposed to the study treatment. Two patients (1 patient [0.5%] in each group) were excluded from mITT population for efficacy analyses due to no post-baseline efficacy data.

**Table 1 Analysis populations - Randomized population**

| | **Placebo (N=195)** | **Lixisenatide (N=196)** | **All (N=391)** |
|---|---|---|---|
| Randomized population | 195 (100%) | 196 (100%) | 391 (100%) |
| Efficacy population Modified Intent-to-Treat (mITT) | 193 (99.0%) | 195 (99.5%) | 388 (99.2%) |
| Safety population | 194 | 196 | 390 |

| | | | |
|---|---|---|---|
| Note: The safety population is tabulated according to treatment actually received (as treated). For the efficacy population, patients are tabulated according to their randomized treatment (as randomized). | | | |

### 6.1.2 Study disposition

Table 2 provides the summary of patient disposition for each treatment group. During the on-treatment period, 27 patients prematurely discontinued the study treatment. The percentage of patients who discontinued the treatment was higher in lixisenatide group than in the placebo group (8.7% and 5.1%, respectively). The main reason for treatment discontinuation was "adverse events" (14 patients) with more patients in the lixisenatide group than those in the placebo group (11 patients [5.6%] and 3 patients [1.5%], respectively), mainly due to AEs from the gastrointerstinal disorders SOC (**Table 20**). The time-to-onset of treatment discontinuation due to any reason for the treatment period is depicted in Figure 8, showing that discontinuation more frequently occurred at the beginning of the study. On Day 181, there were 2 patients in the lixisenatide group, 1 out of 2 patients stopped the treatment and this was recorded as study treatment discontinuation by the investigator.

**Table 2 Patient disposition - Randomized population**

| | **Placebo (N=195)** | **Lixisenatide (N=196)** |
|---|---|---|
| Randomized and not treated | 1 (0.5%) | 0 |
| Subject's request for not treated | 1 (0.5%) | 0 |
| Randomized and not treated | 1 (0.5%) | 0 |
| | | |
| Reason for not treated | 1 (0.5%) | 0 |
| Adverse event | 0 | 0 |
| Lack of efficacy | 0 | 0 |
| Poor compliance to protocol | 0 | 0 |
| Other reasons | 1 (0.5%) | 0 |
| | | |
| Randomized and treated | 194 (99.5%) | 196 (100%) |
| Completed 24-week double-blind treatment period | 184 (94.4%) | 179 (91.3%) |
| Did not complete study treatment | 10 (5.1%) | 17 (8.7%) |
| | | |
| Subject's request for treatment discontinuation | 10 (5.1%) | 16 (8.2%) |
| | | |
| Reason for study treatment discontinuation | 10 (5.1%) | 17 (8.7%) |
| Adverse event | 3 (1.5%) | 11 (5.6%) |
| Lack of efficacy | 1 (0.5%) | 0 |
| Poor compliance to protocol | 1 (0.5%) | 1 (0.5%) |
| Other reasons | 5 (2.6%) | 5 (2.6%) |
| | | |
| Status at last study contact | 195 (100%) | 196 (100%) |
| Alive | 195 (100%) | 196 (100%) |
| Dead | 0 | 0 |

| | | |
|---|---|---|
| Note: Percentages are calculated using the number of randomized patients as denominator. | | |

### 6.1.3 Demographics and baseline characteristics

The demographic and patient baseline characteristics were generally similar between the two treatment groups for the safety population (Table 3). Overall, the median age of the study population was 56 years, the median screening HbA_{1c} was 7.90%, and the median Body Mass Index (BMI) was 26.30 kg/m².

**Table 3 Demographics and patient characteristics at screening or baseline - Safety population**

| | **Placebo (N=194)** | **Lixisenatide (N=196)** | **All (N=390)** |
|---|---|---|---|
| Age (years) | | | |
| Number | 194 | 196 | 390 |
| Mean (SD) | 55.1 (10.5) | 54.5 (10.3) | 54.8 (10.4) |
| Median | 56.0 | 55.0 | 56.0 |
| Min: Max | 21 : 83 | 18 : 75 | 18 : 83 |

| Age group (years) [n (%)] | | | |
|---|---|---|---|
| Number | 194 | 196 | 390 |
| <50 | 53 (27.3%) | 58 (29.6%) | 111 (28.5%) |
| ≥50 to < 65 | 105 (54.1%) | 105 (53.6%) | 210 (53.8%) |
| ≥65 to < 75 | 34 (17.5%) | 32 (16.3%) | 66 (16.9%) |
| ≥75 | 2 (1.0%) | 1 (0.5%) | 3 (0.8%) |
| | | | |

| Gender [n (%)] | | | |
|---|---|---|---|
| Number | 194 | 196 | 390 |
| Male | 91 (46.9%) | 101 (51.5%) | 192 (49.2%) |
| Female | 103 (53.1%) | 95 (48.5%) | 198 (50.8%) |
| | | | |

| Race [n (%)] | | | |
|---|---|---|---|
| Number | 194 | 196 | 390 |
| Caucasian/White | | 0 | 0 |
| Black | 0 | 0 | 0 |
| Asian/Oriental | 194 (100%) | 196 (100%) | 390 (100%) |
| Other | 0 | 0 | 0 |
| | | | |

| Country [n (%)] | | | |
|---|---|---|---|
| Number | 194 | 196 | 390 |
| China | 180 (92.8%) | 178 (90.8%) | 358 (91.8%) |
| Hong Kong | 5 (2.6%) | 13 (6.6%) | 18 (4.6%) |
| Malaysia | 8 (4.1%) | 5 (2.6%) | 13 (3.3%) |
| Thailand | 1 (0.5%) | 0 | 1 (0.3%) |
| | | | |
| Screening HbA_{1c} (%) | | | |
| Number | 194 | 196 | 390 |
| Mean (SD) | 7.99 (0.68) | 8.07 (0.80) | 8.03 (0.74) |
| Median | 7.90 | 7.90 | 7.90 |
| Min : Max | 7.0 : 9.8 | 7.0: 9.9 | 7.0 : 9.9 |
| | | | |

| Randomization strata of screening HbA_{1c} (%) [n (%)] | | | |
|---|---|---|---|
| Number | 194 | 196 | 390 |
| <8 | 100 (51.5%) | 101 (51.5%) | 201 (51.5%) |
| ≥8 | 94 (48.5%) | 95 (48.5%) | 189 (48.5%) |
| | | | |

| Randomization strata of sulfonylurea use at screening [n (%)] | | | |
|---|---|---|---|
| Number | 194 | 196 | 390 |
| Yes | 87 (44.8%) | 89 (45.4%) | 176 (45.1%) |
| No | 107 (55.2%) | 107 (54.6%) | 214 (54.9%) |

| | **Placebo (N=194)** | **Lixisenatide (N=196)** | **All (N=390)** |
|---|---|---|---|
| Age (years) | | | |
| Baseline BMI (kg/m²) | | | |
| Number | 194 | 196 | 390 |
| Mean (SD) | 27.08 (3.75) | 26.75 (3.86) | 26.91 (3.80) |
| Median | 26.58 | 25.96 | 26.30 |
| Min: Max | 20.3 : 40.9 | 20.2 : 38.9 | 20.2 : 40.9 |
| | | | |
| Baseline BMI Categories (kg/m²) [n (%)] | | | |
| Number | 194 | 196 | 390 |
| <25 | 57 (29.4%) | 72 (36.7%) | 129 (33.1%) |
| ≥25 to <30 | 100 (51.5%) | 89 (45.4%) | 189 (48.5%) |
| ≥30 | 37 (19.1%) | 35 (17.9%) | 72 (18.5%) |

| | | | |
|---|---|---|---|
| BMI = Body Mass Index. | | | |

Disease characteristics including diabetic history were generally comparable between the two treatment groups (**Table 4**). Overall, the mean (SD) of duration of T2DM was 6.64 (4.72) years and the mean (SD) age of T2DM onset was 48.1 (9.8) years. Two patients (one in each treatment group) from the same site were reported receiving GLP-1 receptor agonist previously due to participation in a clinical trial on recombinant human GLP-1 (rhGLP-1) at least 8 months before the screening visit.

**Table 4 Disease characteristics at screening or baseline - Safety population**

| | **Placebo (N=194)** | **Lixisenatide (N=196)** | **All (N=390)** |
|---|---|---|---|
| Duration of diabetes (years) | | | |
| Number | 194 | 196 | 390 |
| Mean (SD) | 6.84 (4.80) | 6.45 (4.64) | 6.64 (4.72) |
| Median | 5.62 | 5.52 | 5.58 |
| Min : Max | 0.9 : 21.4 | 1.0 : 25.7 | 0.9 : 25.7 |
| | | | |

| Age at onset of type 2 diabetes (years) | | | |
|---|---|---|---|
| Number | 194 | 196 | 390 |
| Mean (SD) | 48.3 (10.0) | 48.0 (9.6) | 48.1 (9.8) |
| Median | 49.0 | 49.0 | 49.0 |
| Min: Max | 15 : 81 | 15:69 | 15 : 81 |
| | | | |

| History of gestational diabetes [n (%)] | | | |
|---|---|---|---|
| Number (Female) | 103 | 95 | 198 |
| Yes (Female) | 1 (1.0%) | 6 (6.3%) | 7 (3.5%) |
| No (Female) | 102 (99.0%) | 89 (93.7%) | 191 (96.5%) |
| | | | |

| Prior use of GLP-1 receptor agonist [n (%)] | | | |
|---|---|---|---|
| Number | 194 | 196 | 390 |
| Yes | 1 (0.5%) | 1 (0.5%) | 2 (0.5%) |
| No | 193 (99.5%) | 195 (99.5%) | 388 (99.5%) |

| Diabetic retinopathy [n (%)] | | | |
|---|---|---|---|
| Number | 194 | 196 | 390 |
| Yes | 6 (3.1%) | 8 (4.1%) | 14 (3.6%) |
| No | 184 (94.8%) | 186 (94.9%) | 370 (94.9%) |
| Unknown | 4 (2.1%) | 2 (1.0%) | 6 (1.5%) |
| | | | |

| Diabetic sensory or motor neuropathy [n (%)] | | | |
|---|---|---|---|
| Number | 194 | 196 | 390 |
| Yes | 7 (3.6%) | 9 (4.6%) | 16 (4.1%) |
| No | 186 (95.9%) | 184 (93.9%) | 370 (94.9%) |
| Unknown | 1 (0.5%) | 3 (1.5%) | 4 (1.0%) |
| | | | |

| Diabetic autonomic neuropathy [n (%)] | | | |
|---|---|---|---|
| Number | 194 | 196 | 390 |
| Yes | 2 (1.0%) | 3 (1.5%) | 5 (1.3%) |
| No | 190 (97.9%) | 192 (98.0%) | 382 (97.9%) |
| Unknown | 2 (1.0%) | 1 (0.5%) | 3 (0.8%) |
| | | | |

| Diabetic nephropathy [n (%)] | | | |
|---|---|---|---|
| Number | 194 | 196 | 390 |
| Yes | 30 (15.5%) | 26 (13.3%) | 56 (14.4%) |
| Microalbuminuria | 30 (15.5%) | 24 (12.2%) | 54 (13.8%) |
| Overt proteinuria | 0 | 1 (0.5%) | 1 (0.3%) |
| Impaired renal function | 0 | 1 (0.5%) | 1 (0.3%) |
| Dialysis or transplantation | 0 | 0 | 0 |
| No | 160 (82.5%) | 165 (84.2%) | 325 (83.3%) |
| Unknown | 4 (2.1%) | 5 (2.6%) | 9 (2.3%) |
| | | | |

| Categorized albumin/creatinine ratio at randomization [n (%)] | | | |
|---|---|---|---|
| Number | 194 | 194 | 388 |
| <30 µg/mg creatinine (Normal) | 153 (78.9%) | 160 (82.5%) | 313 (80.7%) |
| ≥30 to <300 µg/mg creatinine | | | |
| (Microalbuminuria) | 40 (20.6%) | 33 (17.0%) | 73 (18.8%) |
| ≥300 µg/mg creatinine (Macroalbuminuria) | 1 (0.5%) | 1 (0.5%) | 2 (0.5%) |
| | | | |

| Creatinine clearance at screening (ml/min) | | | |
|---|---|---|---|
| Number | 194 | 196 | 390 |
| Mean (SD) | 113.38 (35.88) | 114.27 (36.59) | 113.83 (36.20) |
| Median | 105.31 | 109.75 | 106.63 |
| Min: Max | 51.9: 253.1 | 32.7: 242.8 | 32.7 : 253.1 |

| Creatinine clearance categories at screening [n (%)] | | | |
|---|---|---|---|
| Number | 194 | 196 | 390 |
| <30 ml/min (severe renal impairment) | 0 | 0 | 0 |
| ≥30 - <50 ml/min (moderate renal impairment) | 0 | 3 (1.5%) | 3 (0.8%) |
| ≥50 - ≤80 ml/min (mild renal impairment) | 27 (13.9%) | 26 (13.3%) | 53 (13.6%) |
| >80 ml/min (no renal impairment) | 167 (86.1%) | 167 (85.2%) | 334 (85.6%) |

| | | | |
|---|---|---|---|
| GLP-1 = Glucagon like peptide-1. Creatinine clearance value is derived using the equation of Cockcroft and Gault. | | | |

The use of background treatment (metformin and/or sulfonylurea) was generally similar between the 2 treatment groups for the safety population (**Table 5** and **Table 6**).

**Table 5 Disease characteristics - Metformin at baseline - Safety population**

| | **Placebo (N=194)** | **Lixisenatide (N=196)** | **All (N=390)** |
|---|---|---|---|
| Duration of metformin treatment (years) | | | |
| Number | 194 | 196 | 390 |
| Mean (SD) | 3.49 (3.14) | 3.98 (3.99) | 3.74(3.60) |
| Median | 2.70 | 2.78 | 2.76 |
| Min : Max | 0.3 : 17.5 | 0.3 : 22.0 | 0.3 : 22.0 |
| | | | |

| Daily dose of metformin at baseline (mg) | | | |
|---|---|---|---|
| Number | 194 | 196 | 390 |
| Mean (SD) | 1363.4 (221.9) | 1369.9 (219.9) | 1366.7 (220.7) |
| Median | 1500.0 | 1500.0 | 1500.0 |
| Min : Max | 1000 : 1500 | 1000 : 1500 | 1000 : 1500 |
| | | | |

| Categorized daily dose of metformin at baseline (mg) [n (%)] | | | |
|---|---|---|---|
| Number | 194 | 196 | 390 |
| <1000 | 0 | 0 | 0 |
| ≥1000 - <1250 | 52 (26.8%) | 51 (26.0%) | 103 (26.4%) |
| ≥1250 - ≤1500 | 142 (73.2%) | 145 (74.0%) | 287 (73.6%) |
| >1500 | 0 | 0 | 0 |

Overall, about half of the patients (174 patients [44.6%]) were receiving sulfonylurea on top of metformin at the screening visit (**Table 6**). The percentage of "Sulfonylurea use at screening" in **Table 6** as recorded in case report form (CRF) is different with "Randomization strata of sulfonylurea use at screening" recorded in the Interactive Voice Response System/Interactive Web Based System (IVRS/IWRS) in **Table 3**. However, the two treatment groups are in general balanced.

**Table 6 Disease characteristics - Sulfonylurea at screening or randomization - Safety population**

| | **Placebo (N=194)** | **Lixisenatide (N=196)** | **ALL (N=390)** |
|---|---|---|---|
| Sulfonylurea use at screening [n (%)] | | | |
| Number | 194 | 196 | 390 |
| Yes | 92 (47.4%) | 82 (41.8%) | 174 (44.6%) |
| No | 102 (52.6%) | 114 (58.2%) | 216 (55.4%) |
| | | | |

| Duration of sulfonylurea treatment (years) | | | |
|---|---|---|---|
| Number | 92 | 82 | 174 |
| Mean (SD) | 2.79 (2.97) | 3.14 (4.25) | 2.95 (3.62) |
| Median | 1.79 | 1.35 | 1.65 |
| Min : Max | 0.3 : 15.4 | 0.3 : 22.0 | 0.3 : 22.0 |
| | | | |

| Sulfonylurea at randomization by INN and formulation [n(%)] | | | |
|---|---|---|---|
| Number | 92 | 82 | 174 |
| Glibenclamide | 4 (4.3%) | 1 (1.2%) | 5 (2.9%) |
| Gliclazide | 17 (18.5%) | 14 (17.1%) | 31 (17.8%) |
| Gliclazide LM | 23 (25.0%) | 27 (32.9%) | 50 (28.7%) |
| Glimepiride | 30 (32.6%) | 24 (29.3%) | 54 (31.0%) |
| Glipizide | 5 (5.4%) | 6 (7.3%) | 11 (6.3%) |
| Glipizide XL | 7 (7.6%) | 3 (3.7%) | 10 (5.7%) |
| Gliquidone | 6 (6.5%) | 7 (8.5%) | 13 (7.5%) |
| | | | |

| Categorized daily dose of sulfonylurea at randomization [n (%)] | | | |
|---|---|---|---|
| Number | 92 | 82 | 174 |
| Daily SU dose <50 % of the maximal recommended dose according to local labelling | 4 (4.3%) | 4 (4.9%) | 8 (4.6%) |
| Daily SU dose ≥50% to <75% of the maximal recommended dose according to local labeling | 77 (83.7%) | 61 (74.4%) | 138 (79.3%) |
| Dailly SU dose ≥75% to ≤100% of the maximal recommended dose according to local labeling | 11 (12.0%) | 15 (18.3%) | 26 (14.9%) |
| Dailly SU dose > 100% of the maximal recommended dose according to local labeling | 0 | 2 (2.4%) | 2 (1.1%) |

| | | | |
|---|---|---|---|
| INN = International Nonproprietary Name | | | |

Baseline efficacy variables were generally comparable between the 2 treatment groups for the safety population (**Table 7**). The study population in the 2 groups was well matched with regard to the baseline glycemic parameters, including HbA_{1c}, 2-hour PPG, FPG, and body weight.

**Table 7 Baseline efficacy variables - Safety population**

| | **Placebo (N=194)** | **Lixisenatide (N=196)** | **All (N=390)** |
|---|---|---|---|
| HbA_{1c} (%) | | | |
| Number | 194 | 196 | 390 |
| Mean (SD) | 7.85 (0.71) | 7.95 (0.81) | 7.90 (0.76) |
| Median | 7.75 | 7.90 | 7.80 |
| Min : Max | 5.4 : 9.7 | 5.4 : 10.1 | 5.4 : 10.1 |
| Weight (kg) | | | |
| Number | 194 | 196 | 390 |
| Mean (SD) | 72.74 (13.64) | 73.18 (13.93) | 72.96 (13.77) |
| Median | 71.00 | 71.00 | 71.00 |
| Min: Max | 49.0 : 121.0 | 46.0: 117.0 | 46.0 : 121.0 |
| | | | |

| FPG (mmol/L) | | | |
|---|---|---|---|
| Number | 194 | 196 | 390 |
| Mean (SD) | 8.74 (1.83) | 8.84 (2.12) | 8.79 (1.98) |
| Median | 8.41 | 8.52 | 8.44 |
| Min: Max | 4.9 : 14.9 | 4.6: 16.0 | 4.6 : 16.0 |
| | | | |

| 2-hour postprandial plasma glucose* (mmol/L) | | | |
|---|---|---|---|
| Number | 130 | 121 | 251 |
| Mean (SD) | 17.19 (4.06) | 16.07 (3.77) | 16.65 (3.95) |
| Median | 17.18 | 16.10 | 16.82 |
| Min: Max | 7.0 : 28.1 | 4.7: 25.7 | 4.7 : 28.1 |
| | | | |

| Glucose excursion* (mmol/L) | | | |
|---|---|---|---|
| Number | 130 | 121 | 251 |
| Mean (SD) | 8.14 (3.11) | 7.12 (3.21) | 7.65 (3.20) |
| Median | 8.13 | 7.38 | 7.72 |
| Min: Max | 0.4 : 15.9 | -8.3 : 13.9 | -8.3 : 15.9 |

| | | | |
|---|---|---|---|
| FPG = Fasting plasma glucose. * For patients in selected sites where the meal challenge test was performed. Glucose excursion = 2-hour postprandial plasma glucose - plasma glucose 30 minutes prior to the meal test before study drug administration. | | | |

### 6.1.4 Dosage and duration

The average treatment exposure was similar between the two treatment groups (159.4 days [22.8 weeks] and 165.5 days [23.6 weeks] for the lixisenatide and placebo groups, respectively) (**Table 8**). Out of 390 patients, 325 patients (83.3%) had 168 +/-5 days of study treatment allowed by protocol (164 patients [83.7%] and 161 patients [83.0%] in the lixisenatide and placebo groups, respectively). One placebo patient did not record the last administration date on CRF page "End of treatment" and hence the duration of exposure was set to missing following the statistical analysis plan (SAP) data handling convention.

**Table 8 Exposure - Safety population**

| | **Placebo (N=194)** | **Lixisenatide (N=196)** |
|---|---|---|
| Cumulative duration of treatment exposure (patient years) | 87.5 | 85.6 |

| Cumulative duration of treatment exposure (patient years) | 87.5 | 85.6 |
|---|---|---|
| Duration of study treatment (days) | | |
| Number | 193 | 196 |
| Mean (SD) | 165.5 (26.5) | 159.4 (36.6) |
| Median | 169.0 | 169.0 |
| Min: Max | 5: 212 | 1 : 181 |
| | | |

| Duration of study treatment by category [n (%)] | | |
|---|---|---|
| Missing duration | 1 (0.5%) | 0 |
| 1-14 days | 3 (1.5%) | 4 (2.0%) |
| 15-28 days | 2 (1.0%) | 5 (2.6%) |
| 29-56 days | 0 | 3 (1.5%) |
| 57-84 days | 0 | 2 (1.0%) |
| 85-168 days | 37 (19.1%) | 45 (23.0%) |
| >168 days | 151 (77.8%) | 137 (69.9%) |
| | | |

| Cumulative duration of study treatment by category [n (%)] | | |
|---|---|---|
| Missing duration | 1 (0.5%) | 0 |
| ≥1 day | 193 (99.5%) | 196 (100%) |
| ≥15 days | 190 (97.9%) | 192 (98.0%) |
| ≥29 days | 188 (96.9%) | 187 (95.4%) |
| ≥57 days | 188 (96.9%) | 184 (93.9%) |
| ≥85 days | 188 (96.9%) | 182 (92.9%) |
| ≥169 days | 151 (77.8%) | 137 (69.9%) |

| | | |
|---|---|---|
| Duration of exposure = (date of the last double-blind investigational product injection - date of the first double-blind investigational product injection) + 1. | | |

More than 90% of patients in the two treatment groups were at the target total daily dose of 20 µg at the end of the double-blind treatment period (**Table 9**).

**Table 9 Number (%) of patients by final total daily dose at the end of the treatment - Safety population**

| **Final dose** | **Placebo (N=194)** | **Lixisenatide (N=196)** |
|---|---|---|
| 10 µg | 3 (1.5%) | 17 (8.7%) |
| 20 µg | 191 (98.5%) | 179 (91.3%) |

| | | |
|---|---|---|
| Dose = Dose of active drug or volume-matched placebo. Note: Percentages are calculated using the number of safety patients as the denominator. | | |

### 6.2 EFFICACY

### 6.2.1 Primary efficacy endpoint

### Main analysis

The pre-specified primary analysis demonstrated that treatment with lixisenatide resulted in a statistically significant decrease in HbA_{1c} from baseline to Week 24 compared with the placebo group (**Table 10**). The LS mean change in HbA_{1c} was 0.83% for the lixisenatide group and - 0.47% for the placebo group; LS mean difference vs. the placebo was -0.36%, 95% CI: -0.551%, -0.162%; pvalue=0.0004. Patients in China demonstrated the same trend (**Table 28**).

**Table 10 Mean change in HbA_{1c} (%) from baseline to Week 24 - mlTT population**

| **HbA_{1c}(%)** | **Placebo (N=193)** | **Lixisenatide (N=195)** |
|---|---|---|
| Baseline | | |
| Number | 188 | 185 |
| Mean (SD) | 7.83(0.70) | 7.95 (0.81) |
| Median | 7.75 | 7.90 |
| Min: Max | 5.4 : 9.7 | 5.4 : 10.1 |
| | | |
| Week 24 (LOCF) | | |
| Number | 188 | 185 |
| Mean (SD) | 7.37 (1.03) | 7.11 (1.14) |
| Median | 7.20 | 6.90 |
| Min: Max | 5.3:13.8 | 5.1 : 11.7 |
| | | |
| Change from baseline to Week 24 (LOCF) | | |
| Number | 188 | 185 |
| Mean (SD) | -0.46 (0.92) | -0.84 (1.02) |
| Median | -0.50 | -0.90 |
| Min: Max | -2.4 : 5.4 | -2.9 : 4.3 |
| LS Mean (SE) ^{a} | -0.47 (0.104) | -0.83 (0.102) |
| | | |
| LS Mean difference (SE) vs. Placebo ^{a} | - | -0.36 (0.099) |
| 95% CI | - | (-0.551 to -0.162) |
| p-value | | 0.0004 |

| | | |
|---|---|---|
| LOCF = Last observation carried forward. ^{a} Analysis of covariance (ANCOVA) model with treatment groups (lixisenatide and placebo), randomization strata of screening HbA_{1c} (<8.0, ≥8.0%), randomization strata of sulfonylurea use at screening (Yes, No), and country (pooled as China and other countries/areas) as fixed effects and baseline HbA_{1c} value as a covariate. Note: The analysis included measurements obtained before the introduction of rescue medication and up to 3 days after the last dose of the double-blind investigational product injection. Patients with both baseline and Week 24 (LOCF) measurements are included. | | |

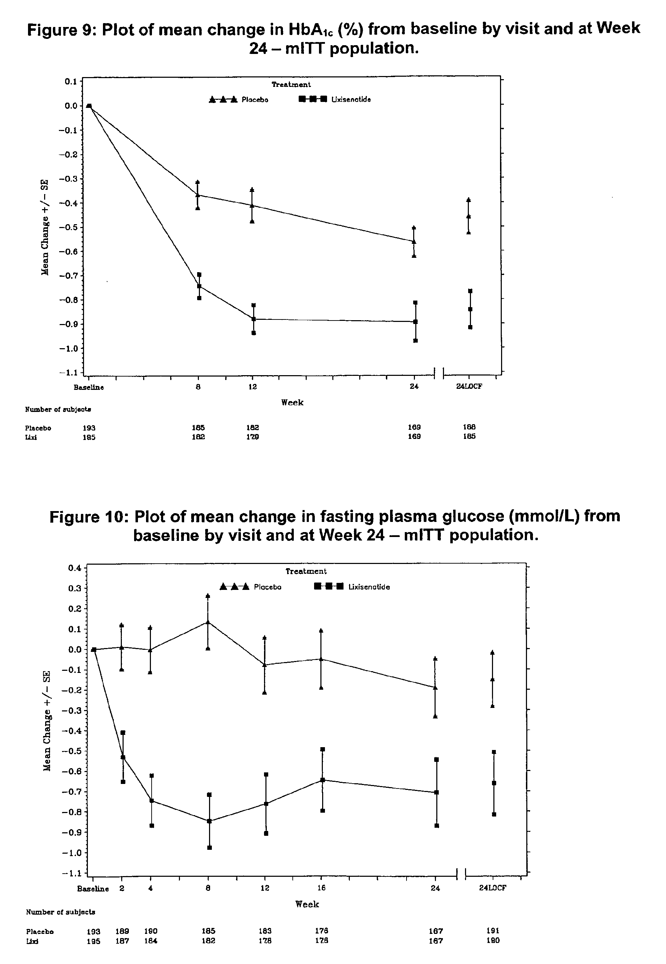

As showed by Figure 9. HbA_{1c} in both treatment groups were already decreased at week 8 and remained reduced during the whole treatment period.

### Secondary analysis

The analysis of HbA_{1c} responder using the CMH method also showed a statistically significant treatment difference between lixisenatide and placebo groups (Table 11). At Week 24, more than half of the patients (53.0%) in the lixisenatide group had achieved HbA_{1c}<7% compared with 38.8% of patients in the placebo group (pvalue=0.0030).

**Table 11 Number (%) of patients with HbA_{1c} value ≤6.5% or <7% respectively at Week 24 - mITT population**

| **HbA_{1c}** (%) | **Placebo (N=193)** | **Lixisenatide (N=195)** |
|---|---|---|
| Number | 188 | 185 |
| ≤6.5% | 34 (18.1%) | 60 (32.4%) |
| >6.5% | 154 (81.9%) | 125 (67.6%) |
| p-value vs. placebo^{a} | - | 0.0010 |
| | | |
| Number | 188 | 185 |
| <7.0% | 73 (38.8%) | 98 (53.0%) |
| ≥7.0% | 115 (61.2%) | 87 (47.0%) |
| p-value vs. placebo^{a} | - | 0.0030 |

| | | |
|---|---|---|
| ^{a} Cochran-Mantel-Haenszel (CMH) method stratified by randomization strata of screening HbA_{1c} (<8.0 or ≥8.0 %) and randomization strata of sulfonylurea use at screening (Yes or No). Note: The analysis included measurements obtained before the introduction of rescue medication and up to 3 days after the last dose of the double-blind investigational product injection. | | |

### 6.2.2 Secondary efficacy endpoints

The results of the 2-hour PPG assessment demonstrated a statistically significant improvement from baseline to Week 24 in lixisenatide group compared with the placebo group (LS mean difference vs. placebo=-4.28 mmol/L; pvalue <0.0001) (Table 12). The analysis of glucose excursion showed similar results (Table 17).

**Table 12 Mean change in 2-hour postprandial plasma glucose (mmol/L) from baseline to Week 24 in selected sites - mITT population**

| **2-hour postprandial plasma glucose (mmol/L)** | **Placebo (N=193)** | **Lixisenatide (N=195)** |
|---|---|---|
| Baseline | | |
| Number | 116 | 107 |
| Mean (SD) | 17.3 (4.16) | 16.27 (3.82) |
| Median | 17.26 | 16.54 |
| Min: Max | 7.0: 28.1 | 4.7 : 25.7 |
| | | |
| Week 24 (LOCF) | | |
| Number | 116 | 107 |
| Mean (SD) | 15.67 (4.13) | 10.94 (4.89) |
| Median | 15.29 | 9.60 |
| Min: Max | 6.7 : 33.1 | 4.0 : 24.3 |
| | | |
| Change from baseline to Week 24 (LOCF) | | |
| Number | 116 | 107 |
| Mean (SD) | -1.67 (3.50) | -5.33 (5.37) |
| Median | -1.64 | -5.50 |
| Min : Max | -11.0: 10.9 | -18.6 : 8.7 |
| LS Mean (SE)^{a} | -1.33 (0.376) | -5.61 (0.393) |
| LS Mean difference (SE) vs. Placebo ^{a} | - | -4.28 (0.548) |
| 95% CI | - | (-5.359 to -3.201) |
| p-value | | <.0001 |

| | | |
|---|---|---|
| LOCF = Last observation carried forward. ^{a} Analysis of covariance (ANCOVA) model with treatment groups (lixisenatide and placebo), randomization strata of screening HbA_{1c} (<8.0, ≥8.0%), randomization strata of sulfonylurea use at screening (Yes, No), and country (pooled as China and other countries/areas) as fixed effects and baseline 2-hour postprandial plasma glucose value as a covariate. Note: The analysis included measurements obtained before the introduction of rescue medication and up to the date of the last dose of the double-blind investigational product injection. Patients with both baseline and Week 24 (LOCF) measurements are included. | | |

For FPG, a statistically significant improvement from baseline to Week 24 was observed in lixisenatide group compared with the placebo group (LS mean difference vs. placebo=-0.48 mmol/L; pvalue=0.0109) (**Table 13**, Figure 10).

**Table 13 Mean change in fasting plasma glucose (mmol/L) from baseline to Week 24 - mITT population**

| **Fasting plasma glucose (mmol/L)** | **Placebo (N=193)** | **Lixisenatide (N=195)** |
|---|---|---|
| Baseline | | |
| Number | 191 | 190 |
| Mean (SD) | 8.75 (1.84) | 8.83(2.12) |
| Median | 8.44 | 8.52 |
| Min: Max | 4.9: 14.9 | 4.6: 16.0 |
| | | |
| Week 24 (LOCF) | | |
| Number | 191 | 190 |
| Mean (SD) | 8.60(2.32) | 8.17(2.12) |
| Median | 8.05 | 7.66 |
| Min : Max | 4.4 : 22.9 | 4.4: 18.7 |
| | | |
| Change from baseline to Week 24 (LOCF) | | |
| Number | 191 | 190 |
| Mean (SD) | -0.15 (1.81) | -0.66 (2.12) |
| Median | -0.22 | -0.64 |
| Min: Max | -7.2: 8.0 | -8.6: 7.1 |
| LS Mean (SE)^{a} | -0.21 (0.200) | -0.69 (0.197) |
| | | |
| LS Mean difference (SE) vs. Placebo^{a} | - | -0.48 (0.187) |
| 95% CI | - | (-0.845 to -0.111) |
| p-value | | 0.0109 |

| | | |
|---|---|---|
| LOCF = Last observation carried forward. ^{a} Analysis of covariance (ANCOVA) model with treatment groups (lixisenatide and placebo), randomization strata of screening HbA_{1c} (<8.0, ≥8.0%), sulfonylurea use at screening (Yes, No), and country (pooled as China and other countries/areas) as fixed effects and baseline fasting plasma glucose as a covariate. Note: The analysis included measurements obtained before the introduction of rescue medication and up to 1 day after the last dose of the double-blind investigational product injection. Patients with both baseline and Week 24 (LOCF) measurements are included. | | |

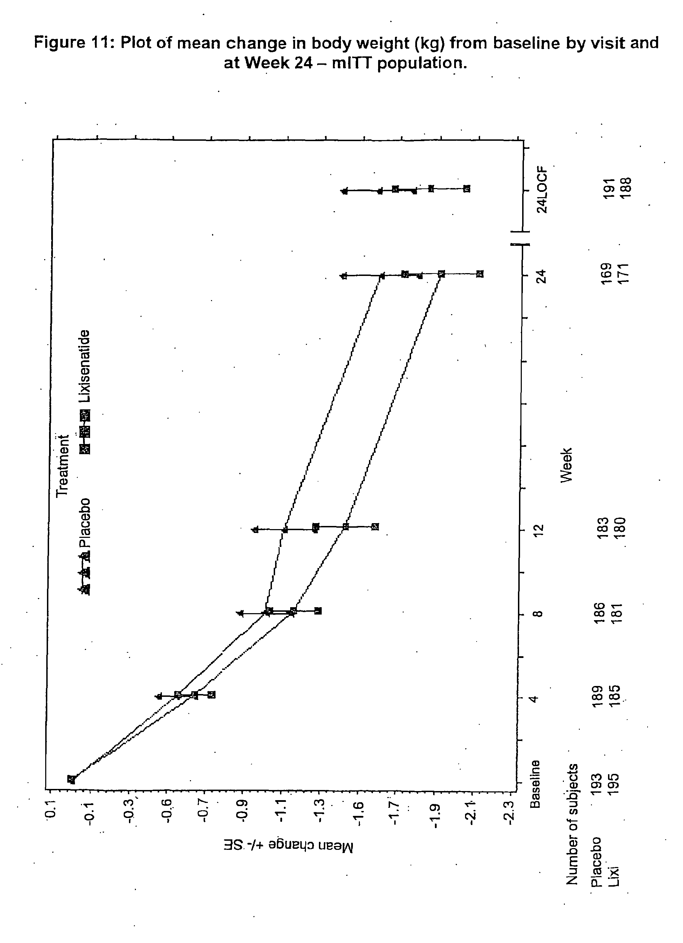

The LS mean body weight loss from baseline at Week 24 was 1.50 kg for the lixisenatide patients and 1.24 kg for the placebo patients (**Table 14**, Figure 11). No statistically significant difference was observed between the two treatment groups (LS mean difference vs. placebo=-0.27 kg; pvalue=0.296). A slightly higher number of patients in the lixisenatide group had a weight loss of 5% or more from baseline to Week 24 compared to the placebo patients (19.7% and 14.7%, respectively) (Table 15).

**Table 14 Mean change in body weight (kg) from baseline to Week 24 - mITT population**

| **Body weight (kg)** | **Placebo (N=193)** | **Lixisenatide (N=195)** |
|---|---|---|
| Baseline | | |
| Number | 191 | 188 |
| Mean (SD) | 72.94 (13.61) | 73.57 (14.03) |
| Median | 71.30 | 72.00 |
| Min : Max | 49.0 : 121.0 | 46.0:117.0 |
| | | |
| Week 24 (LOCF) | | |
| Number | 191 | 188 |
| Mean (SD) | 71.37 (13.27) | 71.72 (13.87) |
| Median | 70.00 | 69.65 |
| Min : Max | 48.0: 120.0 | 45.6: 108.0 |
| | | |
| Change from baseline to Week 24 (LOCF) | | |
| Number | 191 | 188 |
| Mean (SD) | -1.58 (2.57) | -1.85 (2.53) |
| Median | -1.41 | -2.00 |
| Min : Max | -12.0 : 5.0 | -15.0:4.0 |
| LS Mean (SE)^{a} | -1.24 (0.273) | -1.50 (0.267) |
| | | |
| LS Mean difference (SE) vs. Placebo^{a} | - | -0.27 (0.257) |
| 95% CI | - | (-0.776 to 0.237) |
| p-value | | 0.2960 |

| | | |
|---|---|---|
| LOCF = Last observation carried forward. ^{a} Analysis of covariance (ANCOVA) model with treatment groups (lixisenatide and placebo), randomization strata of screening HbA_{1c} (<8.0, ≥8.0%), sulfonylurea use at screening (Yes, No), and country (pooled as China and other countries/areas) as fixed effects and baseline body weight as a covariate. Note: The analysis included measurements obtained before the introduction of rescue medication and up to 3 days after the last dose of the double-blind investigational product injection. Patients with both baseline and Week 24 (LOCF) measurements are included. | | |

**Table 15 Number (%) of patients with >=5 % weight loss from baseline to Week 24 - mITT population**

| **Weight loss** | **Placebo (N=193)** | **Lixisenatide (N=195)** |
|---|---|---|
| Number | 191 | 188 |
| ≥5% | 28 (14.7%) | 37 (19.7%) |
| <5% | 163 (85.3%) | 151 (80.3%) |

| | | |
|---|---|---|
| Note: The analysis included measurements obtained before the introduction of rescue medication and up to 3 days after the last dose of the double-blind investigational product injection. Patients with both baseline and Week 24 (LOCF) measurements are included. | | |

The number of patients requiring rescue therapy at Week 24 was 7 patients [3.6%] in the lixisenatide group compared to 13 patients [6.7%] in the placebo group (Table 16).

**Table 16 Number (%) of patients requiring rescue therapy during the 24-week treatment period - mITT population**

| **Requiring rescue therapy** | **Placebo (N=193)** | **Lixisenatide (N=195)** |
|---|---|---|
| Number | 193 | 195 |
| Yes | 13 (6.7%) | 7 (3.6%) |
| No | 180 (93.3%) | 188 (96.4%) |
| p-value vs. placebo^{a} | - | 0.1580 |

| | | |
|---|---|---|
| ^{a}Cochran-Mantel-Haenszel (CMH) method stratified by randomization strata of screening HbA_{1c} (<8.0 or ≥8.0 %) and sulfonylurea use at screening (Yes, No). | | |

Lixisenatide treatment substantially decreased post prandial plasma glucose excursion from baseline to Week 24 compared to placebo treatment (LS mean difference vs. placebo=-3.99 mmol/L, 95% CI=-4.969, -3.010).

**Table 17 Mean change in glucose excursion from baseline to Week 24 in selected sites - mITT population**

| **Glucose excursion (mmol/L)** | **Placebo (N=193)** | **Lixisenatide (N=195)** |
|---|---|---|
| Baseline | | |
| Number | 116 | 106 |
| Mean (SD) | 8.34 (3.06) | 7.24 (3.32) |
| Median | 8.24 | 7.47 |
| Min: Max | 0.4: 15.9 | -8.3: 13.9 |
| | | |
| Week 24 (LOCF) | | |
| Number | 116 | 106 |
| Mean (SD) | 7.20 (3.34) | 2.76 (4.36) |
| Median | 6.99 | 1.86 |
| Min: Max | -2.0: 14.6 | -6.4: 13.4 |
| Change from baseline to Week 24 (LOCF) | | |
| Number | 116 | 106 |
| Mean (SD) | -1.14 (2.56) | -4.49 (5.23) |
| Median | -1.05 | -4.41 |
| Min: Max | -9.7 : 5.2 | -19.6 : 13.7 |
| LS Mean (SE)^{a} | -0.79 (0.340) | -4.78 (0.356) |
| | | |
| LS Mean difference (SE) vs. Placebo^{a} | - | -3.99 (0.497) |
| 95% CI | - | (-4.969 to -3.010) |

| | | |
|---|---|---|
| LOCF = Last observation carried forward. Glucose excursion = 2-hour postprandial plasma glucose - plasma glucose 30 minutes prior to the meal test before study drug administration. ^{a} Analysis of covariance (ANCOVA) model with treatment groups (lixisenatide and placebo), randomization strata of screening HbA_{1c} (<8.0, ≥8.0%), randomization strata of sulfonylurea use at screening (Yes, No), and country (pooled as China and other countries/areas) as fixed effects and baseline glucose excursion value as a covariate. Note:The analysis included measurements obtained before the introduction of rescue medication and up to the date of the last dose of the double-blind investigational product injection. Patients with both baseline and Week 24 (LOCF) measurements are included. | | |

In summary, the results of the study demonstrated the superior efficacy of treatment with lixisenatide compared with placebo at Week 24 in terms of glycemic control as evidenced by the changes in HbA_{1c}, HbA_{1c} responders, 2-hour PPG, and FPG reduction in patients with T2DM insufficiently controlled by metformin with or without sulfonylurea.

### 6.3 SAFETY

**Table 18** presents the overview summary of patients who had TEAEs. **Table 19** and **Table 20** summarize serious TEAEs, and TEAEs leading to treatment discontinuation by primary SOC, High Level Group Term (HLGT), High Level Term (HLT) and Preferred Term (PT).

As shown in Table 18, the incidence of TEAEs was higher in the lixisenatide group compared to the placebo group (64.3% and 47.4%, respectively). Seven patients had serious TEAEs with a similar incidence rate in the lixisenatide and placebo groups (3 patients [1.5%] and 4 patients [2.1%], respectively). No patient had serious TEAEs leading to death. The percentage of patients with TEAEs leading to treatment discontinuation was higher in the lixisenatide group compared with the placebo group (11 patients [5.6%] and 3 patients [1.5%], respectively), which was mainly related to an imbalance in the gastrointestinal disorders SOC (8 patients [4.1%] in the lixisenatide group and 0 patient in the placebo group, respectively) (Table 20).

Table 26 in the appendix presents the incidences of TEAEs during the on-treatment period occurring in at least 1% of patients in the two treatment groups. Nausea was the most commonly reported TEAE in the lixisenatide group (32 patients [16.3%]) compared with placebo group (5 patients [2.6%]). The second most frequently reported TEAE in the lixisenatide-treated patients was hypoglycemia (18 patients [9.2%]), followed by dizziness (17 patients [8.7%]) and vomiting (15 patients [7.7%]). The corresponding numbers of patients (%) in the placebo group were 9 patients (4.6%) for hypoglycemia, 8 patients (4.1%) for dizziness, and 2 patients (1.0%) for vomiting.

During the on-treatment period, no patient was reported with suspected pancreatitis or at least one value of lipase or amylase ≥3 Upper Limit of Normal range (ULN) in either treatment group.

**Table 18 Overview of adverse event profile: treatment emergent adverse events during the on-treatment period - Safety population**

| | **Placebo (N=194)** | **Lixisenatide (N=196)** |
|---|---|---|
| Patients with any TEAE | 92 (47.4%) | 126 (64.3%) |
| Patients with any serious TEAE | 4 (2.1%) | 3 (1.5%) |
| Patients with any TEAE leading to death | 0 | 0 |
| Patients with any TEAE leading to permanent treatment discontinuation | 3 (1.5%) | 11 (5.6%) |

| | | |
|---|---|---|
| TEAE: Treatment Emergent Adverse Event n (%) = number and percentage of patients with at least one adverse event Note: On-treatment period = the time from the first dose of double-blind investigational product up to 3 days after the last dose administration. | | |

Three patients in the lixisenatide group experienced at least one serious adverse event (SAE) during the on-treatment period (**Table 19**), of which, one patient was reported with 3 events (anaphylactic shock, acute myocardial infarction, and lacunar infarction) after the first injection of lixisenatide and discontinued the treatment permanently; one patient experienced cerebral infarction (day 80 of treatment) which led to treatment discontinuation; one patient experienced abdominal pain with hospitalization (day 13 of treatment), which did not lead to treatment discontinuation. A total of 4 SAEs in 4 patients were reported in the placebo group during the on-treatment period (hypertension, hepatic function abnormal, prostatitis, and benign prostatic hyperplasia, respectively). None of the 4 events were related to IP and led to permanent study treatment discontinuation.

### Anaphylactic shock, acute myocardial infarction, lacunar infarction (156029017):

A 66-year-old male T2DM patient with a history of hypertension, dyslipidemia and no allergy history, experienced allergic-type symptoms 10 minutes after the first dose of IP, with the onset of itchy skin, a rash and flushing all over the body. Ten minutes later, his blood pressure dropped to 60/40 mmHg (with glucose value 10.2 mmol/L) and allergic shock occurred as the patient became unconscious and his breathing became slow and superficial. The patient was hospitalized and treated with dexamethasone, promethazine and dopamine: He recovered from the allergic shock after about 1 hour and recovered from the allergic reaction about 6 hours later. Based on the ECG and cardiac enzymes tested on the same day, he was diagnosed with an acute myocardial infarction without ischemic symptoms. His condition was stable after receiving corrective treatment with clopidogrel, aspirin, low molecular weight heparin, metoprolol, and creatine phosphate. Study treatment was discontinued due to anaphylactic shock and acute myocardial infarction. Six days after permanent IP discontinuation, a brain magnetic resonance imaging (MRI) scan showed lacunar cerebral infarction (was adjudicated by CAC as not being stroke). Two weeks later, the patient recovered and was discharged. There were no residual effects after the lacunar infarction. Case was unblinded by Sanofi (Pharmacovigilance) and the patient had received lixisenatide. The investigator assessed the events of anaphylactic shock and myocardial infarction as related to investigational product, and the lacunar infarction as not related to IP.

### Cerebral infarction (156015018):

A 63-year-old male T2DM patient with a history of hypertension and dyslipidemia was randomized to the lixisenatide treatment group, and experienced mild multiple cerebral infarctions on study treatment (Day 80). Study treatment was discontinued. Four days later, he was hospitalized due to right-sided weakness. A brain MRI scan revealed an acute pontile infarction, multiple brain infarctions, an abnormal signal in the left frontal-temporal region, leukoaraiosis and atherosclerosis. Ten days later, he was discharged in stable condition after corrective treatment (unspecified). However, the patient was re hospitalized due to weakness on the right side of his of body 9 days later. A second brain MRI revealed a pontile infarction, multiple brain infarctions, light brain atrophy, leukoaraiosis and an abnormal signal from the left front frontal lobe considered to be atherosclerosis. Twelve days later, the patient was assessed to be essentially recovered and was discharged after receiving corrective therapy with anti platelet and anti hypertensive therapies. The event was positively adjudicated as non fatal ischemic stroke by CAC. The investigator assessed the cerebral infarction as not related to investigational product.

### Abdominal pain (156008020):

A 65-year-old female T2DM patient with a relevant medical history of breast cancer, uterine fibroids, hypertension, dyslipidemia, angina pectoris, obesity, hyperuricemia, and history of penicillin and food allergies, was randomized to the lixisenatide treatment group. She developed moderate abdominal distending pain 7 days after first dose of IP. She was hospitalized 5 days later for further evaluation and diagnosis of the abdominal pain. Study treatment was continued. Approximately eleven days later, the patient was recovered and discharged without corrective treatment. No further information is available. Admitting diagnosis was still abdominal pain. The investigator assessed the event of abdominal pain as related to the investigational product.

### Hypertension (156007024):

A 63-year-old female T2DM patient with a history of obesity, hypertension, dyslipidemia, myocardial ischemia, and cholelithiasis, was randomized to the placebo treatment group. She experienced moderate hypertension with her blood pressure rising to 200/100 mmHg on study treatment (Day 106). Study treatment was continued. She was hospitalized and received corrective treatment with felodipine, losartan, metoprolol, and simvastatin. Her blood pressure normalized within 3 days and she was discharged 8 days later. The investigator assessed the event as not related to investigational product.

### Hepatic function abnormal (156030011):

A 55-year-old male T2DM patient with a history of fatty liver and dyslipidemia was randomized to the placebo treatment group. He developed severe, medically significant liver dysfunction on study treatment (Day 86). The liver function tests (LFTs) at Visit 9 revealed a serum glutamate pyruvate transaminase (SGPT) 571 U/L (normal: 6-41 U/L), glutamic oxaloacetic transaminase (SGOT) 218 U/L (normal: 9-34 U/L), and gamma glutamyl transferase (GGT) 291 U/L (normal: 11 52 U/L). The patient only felt drowsiness with no nausea or vomiting. He drank alcohol only occasionally and his fatty liver was well controlled. The patient had received both metronidazole and a traditional Chinese medicine for the treatment of periodontitis, which were being used at the time of the liver dysfunction. Study treatment was temporarily stopped for 1 week and the patient received corrective treatment with diammonium glycyrrhizinate. The patient's screening LFTs were normal. At randomization visit, the SGPT had climbed to 94 U/L, the SGOT was 41 U/L, and the GGT was 298 U/L. Twenty-one days later, the patient's LFTs were approximately normal. Study treatment was resumed 6 days later. The investigator assessed the event as not related to investigational product.

### Prostatitis (156015007):

A 62-year-old male T2DM patient with a history of hypertension and prostatitis was randomized to the placebo treatment group. He was hospitalized on Study Day 97 of treatment due to mild abdominal distension that had been ongoing for half a year. Study treatment was continued. Corrective treatment included aztreonam and traditional Chinese medicine. The patient's symptoms improved and he was discharged 11 days later with the diagnosis of chronic prostatitis. The investigator assessed the event as not related to investigational product.

### Benign prostatic hyperplasia (156008016):

A 60-year-old male T2DM patient with a history of hypertension and dyslipidemia was randomized to the placebo treatment group. He experienced moderated prostatic hyperplasia on study treatment (Day 52). The symptoms were characterized by frequency of micturition, urinary retention (for 3 days) and urinary difficulty which had been worsening for 1 year. Study treatment was continued. He was hospitalized, and 8 days later underwent urethral dilation and a transurethral resection of the prostate. Post operatively, he received antiinflammatory medications. Eleven days later, the patient was discharged in stable condition. The investigator assessed the event as not related to investigational product.

**Table 19 Number (%) of patients experiencing serious TEAE(s) presented by primary SOC, HLGT, HLT, and PT - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=194)** | **(N=196)** |
| Any class | 4 (2.1%) | 3 (1.5%) |
| | | |
| IMMUNE SYSTEM DISORDERS | 0 | 1 (0.5%) |
| HLGT: Allergic conditions | 0 | 1 (0.5%) |
| HLT: Anaphylactic responses | 0 | 1 (0.5%) |
| Anaphylactic shock | 0 | 1 (0.5%) |
| | | |
| NERVOUS SYSTEM DISORDERS | 0 | 1 (0.5%) |
| HLGT: Central nervous system vascular disorders | 0 | 1 (0.5%) |
| HLT: Central nervous system haemorrhages and | 0 | 1 (0.5%) |
| cerebrovascular accidents | | |
| Cerebral infarction | 0 | 1 (0.5%) |
| | | |
| CARDIAC DISORDERS | 0 | 1 (0.5%) |
| HLGT: Coronary artery disorders | 0 | 1 (0.5%) |
| HLT: Ischaemic coronary artery disorders | 0 | 1 (0.5%) |
| Acute myocardial infarction | 0 | 1 (0.5%) |
| VASCULAR DISORDERS | 1 (0.5%) | 0 |
| HLGT: Vascular hypertensive disorders | 1 (0.5%) | 0 |
| HLT: Vascular hypertensive disorders NEC | 1 (0.5%) | 0 |
| Hypertension | 1 (0.5%) | 0 |
| | | |
| GASTROINTESTINAL DISORDERS | 0 | 1 (0.5%) |
| HLGT: Gastrointestinal signs and symptoms | 0 | 1 (0.5%) |
| HLT: Gastrointestinal and abdominal pains (excl oral and throat) | 0 | 1 (0.5%) |
| Abdominal pain | 0 | 1 (0.5%) |
| | | |
| HEPATOBILIARY DISORDERS | 1 (0.5%) | 0 |
| HLGT: Hepatic and hepatobiliary disorders | 1 (0.5%) | 0 |
| HLT: Hepatic enzymes and function abnormalities | 1 (0.5%) | 0 |
| Hepatic function abnormal | 1 (0.5%) | 0 |
| | | |
| REPRODUCTIVE SYSTEM AND BREAST DISORDERS | 2 (1.0%) | 0 |
| HLGT: Male reproductive tract infections and inflammations | 1 (0.5%) | 0 |
| HLT: Prostate and seminal vesicles infections and inflammations | 1 (0.5%) | 0 |
| Prostatitis | 1 (0.5%) | 0 |
| HLGT: Prostatic disorders (excl infections and inflammations) | 1 (0.5%) | 0 |
| HLT: Prostatic neoplasms and hypertrophy | 1 (0.5%) | 0 |
| Benign prostatic hyperplasia | 1 (0.5%) | 0 |

| | | |
|---|---|---|
| TEAE: Treatment Emergent Adverse Event, SOC: System Organ Class, HLGT: High Level Group Term, HLT: High Level Term, PT: Preferred Term. MedDRA version: 14.1. n (%) = number and percentage of patients with at least one serious TEAE. Note: On-treatment period = the time from the first dose of double-blind investigational product up to 3 days after the last dose administration. Table sorted by SOC internationally agreed order and HLGT, HLT, PT alphabetic order. | | |

The most common TEAEs leading to treatment discontinuation in the lixisenatide group were gastrointestinal disorders. Eleven (5.6%) lixisenatide-treated patients had permanent treatment discontinuation due to TEAEs and amongst those 8 patients (4.1 %) discontinued treatment due to TEAEs of gastrointestinal disorders (**Table 20**), while none of these gastrointestinal adverse events led to treatment discontinuation in the placebo group. The most frequently reported events leading to treatment discontinuation were nausea and vomiting (3 patients [1.5%] and 2 patients [1.0%], respectively).

**Table 20 Number (%) of patients experiencing TEAE(s) leading to permanent treatment discontinuation by primary SOC, HLGT, HLT, and PT - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=194)** | **(N=196)** |
| Any class | 3 (1.5%) | 11 (5.6%) |
| | | |
| IMMUNE SYSTEM DISORDERS | 0 | 1 (0.5%) |
| HLGT: Allergic conditions | 0 | 1 (0.5%) |
| HLT: Anaphylactic responses | 0 | 1 (0.5%) |
| Anaphylactic shock | 0 | 1 (0.5%) |
| | | |
| NERVOUS SYSTEM DISORDERS | 3 (1.5%) | 2 (1.0%) |
| HLGT: Central nervous system vascular disorders | 0 | 1 (0.5%) |
| HLT: Central nervous system haemorrhages and cerebrovascular accidents | 0 | 1 (0.5%) |
| Cerebral infarction | 0 | 1 (0.5%) |
| HLGT: Headaches | 1 (0.5%) | 0 |
| HLT: Headaches NEC | 1 (0.5%) | 0 |
| Headache | 1 (0.5%) | 0 |
| HLGT: Neurological disorders NEC | 2 (1.0%) | 1 (0.5%) |
| HLT: Disturbances in consciousness NEC | 1 (0.5%) | 0 |
| Somnolence | 1 (0.5%) | 0 |
| HLT: Neurological signs and symptoms NEC | 1 (0.5%) | 1 (0.5%) |
| Dizziness | 1 (0.5%) | 1 (0.5%) |
| HLT: Sensory abnormalities NEC | 1 (0.5%) | 0 |
| Neuralgia | 1 (0.5%) | 0 |
| | | |
| CARDIAC DISORDERS | 0 | 1 (0.5%) |
| HLGT: Coronary artery disorders | 0 | 1 (0.5%) |
| HLT: Ischaemic coronary artery disorders | 0 | 1 (0.5%) |
| Acute myocardial infarction | 0 | 1 (0.5%) |
| | | |
| GASTROINTESTINAL DISORDERS | 0 | 8 (4.1%) |
| HLGT: Gastrointestinal motility and defaecation conditions | 0 | 2 (1.0%) |
| HLT: Diarrhoea (excl infective) | 0 | 1 (0.5%) |
| Diarrhoea | 0 | 1 (0.5%) |
| HLT: Gastrointestinal atonic and hypomotility disorders NEC | 0 | 1 (0.5%) |
| Gastrooesophageal reflux disease | 0 | 1 (0.5%) |
| HLGT: Gastrointestinal signs and symptoms | 0 | 7 (3.6%) |
| HLT: Flatulence, bloating and distension | 0 | 1 (0.5%) |
| Abdominal distension | 0 | 1 (0.5%) |
| HLT: Gastrointestinal signs and symptoms NEC | 0 | 1 (0.5%) |
| Abdominal discomfort | 0 | 1 (0.5%) |
| HLT: Nausea and vomiting symptoms | 0 | 5 (2.6%) |
| Nausea | 0 | 3 (1.5%) |
| Vomiting | 0 | 2 (1.0%) |

| | | |
|---|---|---|
| TEAE: Treatment Emergent Adverse Event, SOC: System Organ Class, HLGT: High Level Group Term, HLT: High Level Term, PT: Preferred Term. MedDRA version: 14.1. n (%) = number and percentage of patients with at least one TEAE leading to permanent treatment discontinuation. Note: On-treatment period = the time from the first dose of double-blind investigational product up to 3 days after the last dose administration. Table sorted by SOC internationally agreed order and HLGT, HLT, PT alphabetic order. | | |

In total, 16 patients (4.1%) had symptomatic hypoglycemia fulfilling the protocol definition during the on-treatment period (11 [5.6%] and 5 [2.6%] in the lixisenatide and placebo groups, respectively) (Table 21). None of these events was considered severe or serious. The numbers of symptomatic hypoglycemia with blood glucose <60 mg/dL were the same in the two treatment groups (3 [1.5%] in each group). Moreover, no difference of the numbers with blood glucose <60 mg/dL between the two treatment groups was observed in patients treated with sulfonylurea on top of metformin (3 patients [3.3%] and 3 patients [3.7%] in the lixisenatide and placebo groups, respectively) (**Table 29**).

**Table 21 Summary of symptomatic hypoglycemia during the on-treatment period - Safety population**

| **Type** | **Placebo (N=194)** | **Lixisenatide (N=196)** |
|---|---|---|
| Total patient years | 89.2 | 87.2 |
| | | |
| Any symptomatic hypoglycemia | | |
| Number of patients with events, n (%) | 5 (2.6%) | 11 (5.6%) |
| Number of patients with events per 100 patient years^{a} | 5.6 | 12.6 |
| Number of events | 9 | 17 |
| Number of events per 100 patient years^{b} | 10.1 | 19.5 |
| | | |
| Blood glucose <60 mg/dL | | |
| Number of patients with events, n (%) | 3 (1.5%) | 3 (1.5%) |
| Number of patients with events per 100 patient years^{a} | 3.4 | 3.4 |
| Number of events | 7 | 7 |
| Number of events per 100 patient years^{b} | 7.8 | 8.0 |
| | | |
| No blood glucose reported | | |
| Number of patients with events, n (%) | 2 (1.0%) | 8 (4.1%) |
| Number of patients with events per 100 patient years^{a} | 2.2 | 9.2 |
| Number of events | 2 | 10 |
| Number of events per 100 patient years^{b} | 2.2 | 11.5 |

| | | |
|---|---|---|
| ^{a} Calculated as (number of patients with events*100 divided by total exposure + 3 days in patient years). ^{b} Calculated as (number of events*100 divided by total exposure + 3 days in patient years). Symptomatic hypoglycemia = Symptomatic hypoglycemia as defined per protocol. Note: On-treatment period = the time from the first dose of double-blind investigational product up to 3 days after the last dose administration. | | |

The injection site reaction events were identified by searching the term "injection site" in either PTs coded from the investigator reported terms or PTs coded from the ARAC diagnosis terms after the allergic reaction adjudication. In total, there were 7 patients (1.8%) who experienced injection site reaction during the on-treatment period (5 on lixisenatide and 2 on placebo), none of which was serious or severe or led to treatment discontinuation (Table 22).

**Table 22 Number (%) of patients experiencing injection site reactions during the on-treatment period - Safety population**

| **Event source** | **Placebo** | **Lixisenatide** |
|---|---|---|
| **Preferred Term** | **(N=194)** | (**N=196**) |
| Any injection site reactions | 2 (1.0%) | 5 (2.6%) |
| | | |
| Investigator reported PTs | 2 (1.0%) | 3 (1.5%) |
| Injection site erythema | 1 (0.5%) | 0 |
| Injection site haemorrhage | 1 (0.5%) | 0 |
| Injection site induration | 1 (0.5%) | 0 |
| Injection site haematoma | 0 | 1 (0.5%) |
| Injection site pruritus | 0 | 1 (0.5%) |
| Injection site reaction | 0 | 1 (0.5%) |
| Injection site vesicles | 0 | 1 (0.5%) |
| | | |
| PTs by ARAC diagnosis | 0 | 4 (2.0%) |
| Injection site reaction | 0 | 4 (2.0%) |

| | | |
|---|---|---|
| ARAC = Allergic Reaction Assessment Committee. Note: On-treatment period = the time from the first dose of double-blind investigational product up to 3 days after the last dose administration. | | |

A total of 15 cases were reported as suspected allergic events by investigators during the on-treatment period and sent to ARAC for adjudication. Three of these cases were adjudicated as allergic reactions by the ARAC (2 patients [1.0%] on lixisenatide and 1 patient [0.5%] on placebo) (**Table 23**). Both events in the lixisenatide group were adjudicated as possibly related to IP by ARAC, out of which 1 event (anaphylactic shock) occurred after the first dose of IP (156029017, see details page 41) and led to permanent treatment discontinuation and 1 event (injection site reaction) occurred after 15 days of treatment (which did not lead to treatment discontinuation) and recovered while patient continued treatment.

**Table 23 Number (%) of patients with events adjudicated as allergic reaction by ARAC during the on-treatment period - Safety population**

| **Relationship to study treatment (by ARAC)** | **MedDRA coded term (PT) for ARAC diagnosis** | **ARAC diagnosis** | **Placebo (N=194)** | **Lixisenatide (N=196)** |
|---|---|---|---|---|
| All | Events adjudicated as an allergic reaction by ARAC | | 1 (0.5%) | 2 (1.0%) |
| | Anaphylactic shock | ANAPHYLACTIC SHOCK | 0 | 1 (0.5%) |
| | Eczema | ECZEMA | 1 (0.5%) | 0 |
| | Injection site reaction | INJECTION SITE REACTION PLUS FLUSH AND GENERALIZED ITCH | 0 | 1 (0.5%) |
| | | | | |
| Possibly related to IP | Events adjudicated as an allergic reaction by ARAC | | 0 | 2 (1.0%) |
| | Anaphylactic shock | ANAPHYLACTIC SHOCK | 0 | 1 (0.5%) |
| | Injection site reaction | INJECTION SITE REACTION PLUS FLUSH AND GENERALIZED ITCH | 0 | 1 (0.5%) |
| | | | | |
| Not related to IP | Events adjudicated as an allergic reaction by ARAC | | 1(0.5%) | 0 |
| | Eczema | ECZEMA | 1 (0.5%) | 0 |

| | | | | |
|---|---|---|---|---|
| ARAC = Allergic Reaction Assessment Committee. IP=Investigational Product. PT=Preferred Term. Note: On-treatment period = the time from the first dose of double-blind investigational product up to 3 days after the last dose administration. | | | | |

No increase of serum Calcitonin was reported as TEAE ( **Table 24**). In the lixisenatide group, one patient was found with calcitonin (23.6 pg/mL) greater than 20 ng/ml at Visit 12 (end of treatment visit), however, the re-test calcitonin value returned to normal (15.7 pg/mL) within 1 week. No AE was reported for this case.

**Table 24 Serum calcitonin - Number (%) of patients by pre-defined categories during the on-treatment period according to baseline category-Safety population**

| **Laboratory criteria** | | |
|---|---|---|
| **Baseline status** | **Placebo** | **Lixisenatide** |
| **Post-baseline** | **(N=194)** | **(N=196)** |
| Calcitonin (ng/L) | | |
| Total* | | |
| ≤ULN | 185/185 (100%) | 183/184 (99.5%) |
| >ULN - <20 ng/L | 0/185 | 0/184 |
| ≥20 ng/L - <50 ng/L | 0/185 | 1/184 (0.5%) |
| ≥50 ng/L | 0/185 | 0/184 |
| Missing | | |
| ≤ULN | 0/0 | 0/0 |
| >ULN - <20 ng/L | 0/0 | 0/0 |
| ≥20 ng/L - <50 ng/L | 0/0 | 0/0 |
| ≥50 ng/L | 0/0 | 0/0 |
| | | |
| ≤ULN | | |
| ≤ULN | 185/185 (100%) | 183/184 (99.5%) |
| >ULN - <20 ng/L | 0/185 | 0/184 |
| ≥20 ng/L - <50 ng/L | 0/185 | 1/184 (0.5%) |
| ≥50 ng/L | 0/185 | 0/184 |
| | | |
| >ULN - <20 ng/L | | |
| ≤ULN | 0/0 | 0/0 |
| >ULN - <20 ng/L | 0/0 | 0/0 |
| ≥20 ng/L - <50 ng/L | 0/0 | 0/0 |
| ≥50 ng/L | 0/0 | 0/0 |
| | | |
| ≥20 ng/L - <50 ng/L | | |
| ≤ULN | 0/0 | 0/0 |
| >ULN - <20 ng/L | 0/0 | 0/0 |
| ≥20 ng/L - <50 ng/L | 0/0 | 0/0 |
| ≥50 ng/L | 0/0 | 0/0 |
| | | |
| ≥50 ng/L | | |
| ≤ULN | 0/0 | 0/0 |
| >ULN - <20 ng/L | 0/0 | 0/0 |
| ≥20 ng/L - <50 ng/L | 0/0 | 0/0 |
| ≤50 ng/L | 0/0 | 0/0 |

| | | |
|---|---|---|
| ULN= Upper limit of normal *Regardless of baseline. Note: On-treatment period = the time from the first dose of double-blind investigational product up to 3 days after the last dose administration. The numerator represents the number of patients who were in the pre-specified categories at post-baseline in each baseline category. The denominator for each parameter within a treatment group is the number of patients for the treatment group who had that parameter assessed post-baseline by baseline status. A patient is counted only in the worst category. | | |

In conclusion, lixisenatide was well tolerated during the 24-week treatment period. Incidences of TEAEs leading to permanent discontinuation of study treatment were higher in the lixisenatide group than in the placebo group and this was mainly due to gastrointestinal TEAEs. Incidences of serious TEAEs were similar in the two treatment groups. A higher percentage of patients treated with lixisenatide compared with placebo experienced symptomatic hypoglycemia, while the numbers of patients with symptomatic hypoglycemia with blood glucose <60 mg/dL were exactly the same in the two treatment groups. Nausea, dizziness and vomiting were reported more frequently with lixisenatide than with placebo. No new specific safety concern was observed during the trial.

### 7 APPENDIX

**Table 25 Mean change in HbA_{1c} (%) from baseline by visit - mITT population**

| | **Observed data** | | | | | | | **Change from baseline** | | | | | | |
|---|---|---|---|---|---|---|---|---|---|---|---|---|---|---|
| **Treatment** | | | | | | | | | | | | | | |
| **Time point** | **N** | **Mean** | **SD** | **SE** | **Median** | **Min** | **Max** | **N** | **Mean** | **SD** | **SE** | **Median** | **Min** | **Max** |
| Placebo (N=193) | | | | | | | | | | | | | | |
| Screening | 193 | 7.98 | 0.68 | 0.049 | 7.90 | 7.0 | 9.8 | | | | | | | |
| Baseline | 193 | 7.84 | 0.71 | 0.051 | 7.70 | 5.4 | 9.7 | | | | | | | |
| Week 8 | 185 | 7.46 | 0.88 | 0.065 | 7.40 | 5.3 | 13.0 | 185 | -0.37 | 0.73 | 0.054 | -0.30 | -2.3 | 4.6 |
| Week 12 | 182 | 7.41 | 0.97 | 0.072 | 7.30 | 5.2 | 13.7 | 182 | -0.41 | 0.88 | 0.065 | -0.40 | -2.6 | 5.3 |
| Week 24 | 169 | 7.25 | 0.86 | 0.066 | 7.10 | 5.3 | 10.3 | 169 | -0.56 | 0.76 | 0.058 | -0.60 | -2.3 | 2.2 |
| Week 24(LOCF) | 188 | 7.37 | 1.03 | 0.075 | 7.20 | 5.3 | 13.8 | 188 | -0.46 | 0.92 | 0.067 | -0.50 | -2.4 | 5.4 |
| | | | | | | | | | | | | | | |
| Lixisenatide (N=195) | | | | | | | | | | | | | | |
| Screening | 195 | 8.07 | 0.80 | 0.057 | 7.90 | 7.0 | 9.9 | | | | | | | |
| Baseline | 195 | 7.95 | 0.81 | 0.058 | 7.90 | 5.4 | 10.1 | | | | | | | |
| Week 8 | 182 | 7.21 | 0.93 | 0.069 | 7.00 | 5.2 | 11.0 | 182 | -0.75 | 0.65 | 0.049 | -0.70 | -2.3 | 1.8 |
| Week 12 | 179 | 7.07 | 0.93 | 0.070 | 6.90 | 5.1 | 11.5 | 179 | -0.88 | 0.77 | 0.058 | -0.90 | -2.8 | 1.7 |
| Week 24 | 169 | 7.05 | 1.10 | 0.085 | 6.90 | 5.1 | 11.7 | 169 | -0.89 | 1.02 | 0.078 | -1.00 | -2.9 | 4.3 |
| Week 24(LOCF) | 185 | 7.11 | 1.14 | 0.084 | 6.90 | 5.1 | 11.7 | 185 | -0.84 | 1.02 | 0.075 | -0.90 | -2.9 | 4.3 |

| | | | | | | | | | | | | | | |
|---|---|---|---|---|---|---|---|---|---|---|---|---|---|---|
| LOCF = Last observation carried forward. Note: The analysis excluded measurements obtained after the introduction of rescue medication and/or after the treatment cessation plus 3 days. | | | | | | | | | | | | | | |

**Table 26 Number (%) of patients experiencing common TEAE(s) (PT ≥1% in any treatment group) presented by primary SOC, HLGT, HLT and PT - Safety population**

| **PRIMARY SYSTEM ORGAN CLASS** | | |
|---|---|---|
| **HLGT: High Level Group Term** | | |
| **HLT: High Level Term** | **Placebo** | **Lixisenatide** |
| **Preferred Term** | **(N=194)** | **(N=196)** |
| Any class | 92 (47.4%) | 126 (64.3%) |
| | | |
| INFECTIONS AND INFESTATIONS | 19 (9.8%) | 28 (14.3%) |
| HLGT: Infections - pathogen unspecified | 17 (8.8%) | 26 (13.3%) |
| HLT: Upper respiratory tract infections | 14 (7.2%) | 21 (10.7%) |
| Nasopharyngitis | 5 (2.6%) | 9 (4.6%) |
| Upper respiratory tract infection | 8 (4.1%) | 12 (6.1%) |
| HLT: Urinary tract infections | 2 (1.0%) | 2 (1.0%) |
| Urinary tract infection | 2 (1.0%) | 2 (1.0%) |
| | | |
| BLOOD AND LYMPHATIC SYSTEM DISORDERS | 1 (0.5%) | 7 (3.6%) |
| HLGT: Anaemias nonhaemolytic and marrow depression | 0 | 5 (2.6%) |
| HLT: Anaemias NEC | 0 | 5 (2.6%) |
| Anaemia | 0 | 5 (2.6%) |
| | | |
| METABOLISM AND NUTRITION DISORDERS | 27 (13.9%) | 34 (17.3%) |
| HLGT: Appetite and general nutritional disorders | 1 (0.5%) | 4 (2.0%) |
| HLT: Appetite disorders | 1 (0.5%) | 4 (2.0%) |
| Decreased appetite | 1 (0.5%) | 3 (1.5%) |
| HLGT: Glucose metabolism disorders (incl diabetes mellitus) | 9 (4.6%) | 18 (9.2%) |
| HLT: Hypoglycaemic conditions NEC | 9 (4.6%) | 18 (9.2%) |
| Hypoglycaemia | 9 (4.6%) | 18 (9.2%) |
| HLGT: Lipid metabolism disorders | 7 (3.6%) | 6 (3.1%) |
| HLT: Hyperlipidaemias NEC | 4(2.1%) | 2 (1.0%) |
| Hyperlipidaemia | 4 (2.1%) | 2 (1.0%) |
| HLT: Lipid metabolism and deposit disorders NEC | 3 (1.5%) | 3 (1.5%) |
| Dyslipidaemia | 3 (1.5%) | 3 (1.5%) |
| HLGT: Purine and pyrimidine metabolism disorders | 11 (5.7%) | 7 (3.6%) |
| HLT: Disorders of purine metabolism | 11 (5.7%) | 7 (3.6%) |
| Hyperuricaemia | 10 (5.2%) | 7 (3.6%) |
| | | |
| NERVOUS SYSTEM DISORDERS | 15 (7.7%) | 24 (12.2%) |
| HLGT: Headaches | 2 (1.0%) | 5 (2.6%) |
| HLT: Headaches NEC | 2 (1.0%) | 5 (2.6%) |
| Headache | 2 (1.0%) | 5 (2.6%) |
| Any class | 92 (47.4%) | 126 (64.3%) |
| HLGT: Neurological disorders NEC | 9 (4.6%) | 20 (10.2%) |
| HLT: Disturbances in consciousness NEC | 1 (0.5%) | 4 (2.0%) |
| Lethargy | 0 | 2 (1.0%) |
| Somnolence | 1 (0.5%) | 2 (1.0%) |
| HLT: Neurological signs and symptoms NEC | 8 (4.1%) | 17 (8.7%) |
| Dizziness | 8 (4.1%) | 17 (8.7%) |
| HLT: Sensory abnormalities NEC | 1 (0.5%) | 3 (1.5%) |
| Hypoaesthesia | 0 | 3 (1.5%) |
| | | |
| CARDIAC DISORDERS | 4 (2.1%) | 3 (1.5%) |
| HLGT: Cardiac disorder signs and symptoms | 2 (1.0%) | 2 (1.0%) |
| HLT: Cardiac signs and symptoms NEC | 2 (1.0%) | 2 (1.0%) |
| Palpitations | 2 (1.0%) | 2 (1.0%) |
| HLGT: Coronary artery disorders | 2 (1.0%) | 1 (0.5%) |
| HLT: Coronary artery disorders NEC | 2 (1.0%) | 0 |
| Coronary artery disease | 2 (1.0%) | 0 |
| | | |
| VASCULAR DISORDERS | 8 (4.1%) | 1 (0.5%) |
| HLGT: Vascular hypertensive disorders | 8 (4.1%) | 1 (0.5%) |
| HLT: Vascular hypertensive disorders NEC | 8 (4.1%) | 1 (0.5%) |
| Hypertension | 8 (4.1%) | 1 (0.5%) |
| | | |
| GASTROINTESTINAL DISORDERS | 18 (9.3%) | 57(29.1%) |
| HLGT: Dental and gingival conditions | 4 (2.1%) | 3 (1.5%) |
| HLT: Dental and periodontal infections and inflammations | 2 (1.0%) | 2 (1.0%) |
| Periodontitis | 2 (1.0%) | 1 (0.5%) |
| HLT: Dental pain and sensation disorders | 2 (1.0%) | 1 (0.5%) |
| Toothache | 2 (1.0%) | 1 (0.5%) |
| HLGT: Gastrointestinal motility and defaecation conditions | 4 (2.1%) | 11 (5.6%) |
| HLT: Diarrhoea (excl infective) | 2 (1.0%) | 7 (3.6%) |
| Diarrhoea | 2 (1.0%) | 7 (3.6%) |
| HLT: Gastrointestinal atonic and hypomotility disorders NEC | 2 (1.0%) | 4 (2.0%) |
| Constipation | 2 (1.0%) | 1 (0.5%) |
| Gastrooesophageal reflux disease | 0 | 2 (1.0%) |
| HLGT: Gastrointestinal signs and symptoms | 11 (5.7%) | 47 (24.0%) |
| HLT: Flatulence, bloating and distension | 1 (0.5%) | 6 (3.1%) |
| Abdominal distension | 1 (0.5%) | 6(3.1%) |
| Any class | 92 (47.4%) | 126 (64.3%) |
| HLT: Gastrointestinal and abdominal pains (excl oral and throat) | 1 (0.5%) | 4 (2.0%) |
| Abdominal pain | 1 (0.5%) | 2 (1.0%) |
| Abdominal pain upper | 0 | 2 (1.0%) |
| HLT: Gastrointestinal signs and symptoms NEC | 1 (0.5%) | 3 (1.5%) |
| Abdominal discomfort | 1 (0.5%) | 3 (1.5%) |
| HLT: Nausea and vomiting symptoms | 7 (3.6%) | 42 (21.4%) |
| Nausea | 5 (2.6%) | 32 (16.3%) |
| Regurgitation | 0 | 3 (1.5%) |
| Vomiting | 2 (1.0%) | 15 (7.7%) |
| | | |
| SKIN AND SUBCUTANEOUS TISSUE DISORDERS | 7 (3.6%) | 9 (4.6%) |
| HLGT: Epidermal and dermal conditions | 6 (3.1 %) | 7 (3.6%) |
| HLT: Dermatitis and eczema | 3 (1.5%) | 2 (1.0%) |
| Dermatitis allergic | 0 | 2 (1.0%) |
| Eczema | 3 (1.5%) | 0 |
| HLT: Pruritus NEC | 3 (1.5%) | 3 (1.5%) |
| Pruritus | 3 (1.5%) | 3 (1.5%) |
| HLT: Rashes, eruptions and exanthems NEC | 0 | 2 (1.0%) |
| Rash | 0 | 2 (1.0%) |
| | | |
| MUSCULOSKELETAL AND CONNECTIVE TISSUE DISORDERS | 8 (4.1%) | 5 (2.6%) |
| HLGT: Muscle disorders | 3 (1.5%) | 1 (0.5%) |
| HLT: Muscle related signs and symptoms NEC | 3 (1.5%) | 0 |
| Muscle spasms | 3 (1.5%) | 0 |
| HLGT: Musculoskeletal and connective tissue disorders NEC | 3 (1.5%) | 3 (1.5%) |
| HLT: Musculoskeletal and connective tissue pain and | | |
| discomfort | 3 (1.5%) | 3 (1.5%) |
| Musculoskeletal pain | 2 (1.0%) | 1 (0.5%) |
| | | |
| RENAL AND URINARY DISORDERS | 24 (12.4%) | 23 (11.7%) |
| HLGT: Nephropathies | 13 (6.7%) | 13 (6.6%) |
| HLT: Nephropathies and tubular disorders NEC | 13 (6.7%) | 13 (6.6%) |
| Diabetic nephropathy | 13 (6.7%) | 13 (6.6%) |
| HLGT: Renal disorders (excl nephropathies) | 2 (1.0%) | 4 (2.0%) |
| HLT: Renal failure and impairment | 2 (1.0%) | 4 (2.0%) |
| Renal impairment | 2 (1.0%) | 4 (2.0%) |
| Any class | 92 (47.4%) | 126 (64.3%) |
| HLGT: Urinary tract signs and symptoms | 8 (4.1%) | 6 (3.1%) |
| HLT: Urinary abnormalities | 8 (4.1%) | 5 (2.6%) |
| Microalbuminuria | 7 (3.6%) | 3 (1.5%) |
| Proteinuria | 1 (0.5%) | 2 (1.0%) |
| | | |
| GENERAL DISORDERS AND ADMINISTRATION SITE CONDITIONS | 7 (3.6%) | 10 (5.1%) |
| HLGT: General system disorders NEC | 4 (2.1%) | 5 (2.6%) |
| HLT: Asthenic conditions | 2 (1.0%) | 5 (2.6%) |
| Asthenia | 0 | 3 (1.5%) |
| Fatigue | 2 (1.0%) | 3 (1.5%) |
| | | |
| INVESTIGATIONS | 17 (8.8%) | 19 (9.7%) |
| HLGT: Lipid analyses | 3 (1.5%) | 2 (1.0%) |
| HLT: Lipoprotein and lipid tests NEC | 0 | 2 (1.0%) |
| Lipids increased | 0 | 2 (1.0%) |
| HLT: Triglyceride analyses | 2 (1.0%) | 0 |
| Blood triglycerides increased | 2 (1.0%) | 0 |
| HLGT: Metabolic, nutritional and blood gas investigations | 6 (3.1%) | 11 (5.6%) |
| HLT: Carbohydrate tolerance analyses (incl diabetes) | 5 (2.6%) | 10 (5.1%) |
| Blood glucose decreased | 5 (2.6%) | 10 (5.1%) |
| | | |
| INJURY, POISONING AND PROCEDURAL COMPLICATIONS | 2 (1.0%) | 6 (3.1%) |
| HLGT: Injuries NEC | 2 (1.0%) | 3 (1.5%) |
| HLT: Muscle, tendon and ligament injuries | 2 (1.0%) | 1 (0.5%) |
| Ligament sprain | 2(1.0%) | 1 (0.5%) |

| | | |
|---|---|---|
| TEAE: Treatment Emergent Adverse Event, SOC: System Organ Class, HLGT: High Level Group Term, HLT: High Level Term, PT: Preferred Term. MedDRA version: 14.1. n (%) = number and percentage of patients with at least one TEAE. Note: On-treatment period = the time from the first dose of double-blind investigational product up to 3 days after the last dose administration. Table sorted by SOC internationally agreed order and HLGT, HLT, PT by alphabetic order. Only SOC with at least one PT ≥1% in at least one group are presented. | | |

**Table 27 Number (%) of patients by final total daily dose at the end of the titration - Safety population**

| **Dose at the end of titration** | **Placebo (N=194)** | **Lixisenatide (N=196)** |
|---|---|---|
| 10 µg | 5 (2.6%) | 8 (4.1%) |
| 20 µg | 189 (97.4%) | 188 (95.9%) |

| | | |
|---|---|---|
| Dose = Dose of active drug or volume-matched placebo. The scheduled visit for end of titration per protocol would be Visit 5/Week 2. Note: Percentages are calculated using the number of safety patients as the denominator. | | |

**Table 28 Mean change in HbA_{1c} (%) from baseline to Week 24 - mITT population in China**

| **HbA_{1c} (%)** | **Placebo (N=179)** | **Lixisenatide (N=177)** |
|---|---|---|
| Baseline | | |
| Number | 174 | 168 |
| Mean (SD) | 7.86(0.71) | 7.98 (0.81) |
| Median | 7.80 | 7.90 |
| Min: Max | 5.4 : 9.7 | 5.4 : 10.1 |
| | | |
| Week 24 (LOCF) | | |
| Number | 174 | 168 |
| Mean (SD) | 7.38 (1.04) | 7.14 (1.18) |
| Median | 7.20 | 6.90 |
| Min: Max | 5.3: 13.8 | 5.1 : 11.7 |
| | | |
| Change from baseline to Week 24 (LOCF) | | |
| Number | 174 | 168 |
| Mean (SD) | -0.48 (0.93) | -0.84 (1.05) |
| Median | -0.60 | -1.00 |
| Min: Max | -2.4 : 5.4 | -2.9 : 4.3 |
| LS Mean (SE) ^{a} | -0.50 (0.074) | -0.83 (0.075) |
| | | |
| LS Mean difference (SE) vs. Placebo ^{a} | - | -0.33 (0.106) |
| 95% CI | - | (-0.541 to -0.126) |
| p-value | | 0.0017 |

| | | |
|---|---|---|
| LOCF = Last observation carried forward. ^{a} Analysis of covariance (ANCOVA) model with treatment groups (lixisenatide and placebo), randomization strata of screening HbA_{1c} (<8.0, ≥8.0%) and randomization strata of sulfonylurea use at screening (Yes, No) as fixed effects and baseline HbA_{1c} value as a covariate. Note: The analysis included measurements obtained before the introduction of rescue medication and up to 3 days after the last dose of the double-blind investigational product injection. Patients with both baseline and Week 24 (LOCF) measurements are included. | | |

**Table 29 Summary of symptomatic hypoglycemia by sulfonylurea use at screening during the on-treatment period - Safety population**

| | **Sulfonylurea use at screening: Yes** | | **Sulfonylurea use at screening: No** | |
|---|---|---|---|---|
| **Type** | **Placebo (N=92)** | **Lixisenatide (N=82)** | **Placebo (N=102)** | **Lixisenatide (N=114)** |
| Total patient years | 42.36 | 36.44 | 46.80 | 50.73 |
| Any symptomatic hypoglycemia | | | | |
| Number of patients with events, n (%) | 5 (5.4%) | 7 (8.5%) | 0 | 4 (3.5%) |
| Number of patients with events per 100 patient years^{a} | 11.8 | 19.2 | 0 | 7.9 |
| Number of events | 9 | 12 | 0 | 5 |
| Number of events per 100 patient years^{b} | 21.2 | 32.9 | 0 | 9.9 |
| | | | | |
| Blood glucose <60 mg/dL | | | | |
| Number of patients with events, n (%) | 3 (3.3%) | 3 (3.7%) | 0 | 0 |
| Number of patients with events per 100 patient years^{a} | 7.1 | 8.2 | 0 | 0 |
| Number of events | 7 | 7 | 0 | 0 |
| Number of events per 100 patient years^{b} | 16.5 | 19.2 | 0 | 0 |
| | | | | |
| No blood glucose reported | | | | |
| Number of patients with events, n (%) | 2 (2.2%) | 4 (4.9%) | 0 | 4 (3.5%) |
| Number of patients with events per 100 patient years^{a} | 4.7 | 11 | 0 | 7.9 |
| Number of events | 2 | 5 | 0 | 5 |
| Number of events per 100 patient years^{b} | 4.7 | 13.7 | 0 | 9.9 |

| | | | | |
|---|---|---|---|---|
| ^{a} Calculated as (number of patients with events*100 divided by total exposure + 3 days in patient years). ^{b} Calculated as (number of events*100 divided by total exposure + 3 days in patient years). Symptomatic hypoglycemia = Symptomatic hypoglycemia as defined per protocol. Note: On-treatment period = the time from the first dose of double-blind investigational product up to 3 days after the last dose administration. | | | | |

## Claims

1. A pharmaceutical combination for use in the reduction of plasma glucagon level in diabetes type 2 patients, said combination comprising
(a) desPro³⁶Exendin-4(1-39)-Lys₆-NH₂ or/and a pharmaceutically acceptable salt thereof,
(b) metformin or/and a pharmaceutically acceptable salt thereof, and
(c) a sulfonyl urea,
wherein the subject to be treated has a 2 hours postprandial plasma glucose concentration of at least 14 mmol/L.

2. The pharmaceutical combination for use of claim 1, wherein the subject to be treated is obese.

3. The pharmaceutical combination for use of any one of the preceding claims, wherein the subject to be treated has a body mass index of at least 30 kg/m².

4. The pharmaceutical combination for use of any one of the preceding claims, wherein the subject to be treated is an adult subject.

5. The pharmaceutical combination for use of any one of the preceding claims, wherein in the subject to be treated, diabetes mellitus type 2 has been diagnosed at least 1 year or at least 2 years before onset of therapy.

6. The pharmaceutical combination for use of any one of the preceding claims, wherein the subject to be treated has a HbA_{1c} value of about 7 to about 10%.

7. The pharmaceutical combination for use of any one of the preceding claims, wherein the subject to be treated has a fasting plasma glucose concentration of at least 8 mmol/L.

8. The pharmaceutical combination for use of any one of the preceding claims, wherein the subject to be treated has a glucose excursion of at least 2 mmol/L, at least 3 mmol/L, at least 4 mmol/L or at least 5 mmol/L, wherein the glucose excursion is the difference of the 2 hours postprandial plasma glucose concentration and plasma glucose concentration 30 minutes prior to a meal test.

9. The pharmaceutical combination for use of any one of the preceding claims, wherein the subject to be treated is of Oriental or/and Asian race.

10. The pharmaceutical combination for use of any one of the preceding claims, wherein the desPro³⁶Exendin-4(1-39)-Lys₆-NH₂ or/and the pharmaceutically acceptable salt thereof is prepared for parenteral administration.

11. The pharmaceutical combination for use of any one of the preceding claims, wherein the desPro³⁶Exendin-4(1-39)-Lys₆-NH₂ or/and the pharmaceutically acceptable salt thereof is prepared for administration in a daily dose selected from the range of 10 µg to 20 µg.

12. The pharmaceutical combination for use of any one of the preceding claims, wherein the metformin or/and the pharmaceutically acceptable salt thereof is prepared for oral administration.

13. The pharmaceutical combination for use of any one of the preceding claims, wherein the sulfonyl urea is prepared for oral administration.

## Patentansprüche

1. Pharmazeutische Kombination zur Verwendung beim Absenken des Plasmaglucagonspiegels bei Patienten mit Diabetes vom Typ 2, wobei die Kombination Folgendes umfasst:
(a) desPro³⁶Exendin-4-(1-39)-Lys₆-NH₂ und/oder ein pharmazeutisch unbedenkliches Salz davon,
(b) Metformin und/oder ein pharmazeutisch unbedenkliches Salz davon und
(c) einen Sulfonylharnstoff,
wobei der zu behandelnde Patient 2 Stunden nach einer Mahlzeit eine Plasmaglucosekonzentration von mindestens 14 mmol/l aufweist.

2. Pharmazeutische Kombination zur Verwendung nach Anspruch 1, wobei der zu behandelnde Patient adipös ist.

3. Pharmazeutische Kombination zur Verwendung nach einem der vorhergehenden Ansprüche, wobei der zu behandelnde Patient einen Körpermasseindex von mindestens 30 kg/m² hat.

4. Pharmazeutische Kombination zur Verwendung nach einem der vorhergehenden Ansprüche, wobei es sich bei dem zu behandelnden Patienten um einen erwachsenen Patienten handelt.

5. Pharmazeutische Kombination zur Verwendung nach einem der vorhergehenden Ansprüche, wobei bei dem zu behandelnden Patienten Diabetes mellitus vom Typ 2 mindestens 1 Jahr oder mindestens 2 Jahre vor dem Beginn der Therapie diagnostiziert wurde.

6. Pharmazeutische Kombination zur Verwendung nach einem der vorhergehenden Ansprüche, wobei der zu behandelnde Patient einen HbA_{1c}-Wert von etwa 7 bis etwa 10% hat.

7. Pharmazeutische Kombination zur Verwendung nach einem der vorhergehenden Ansprüche, wobei der zu behandelnde Patient eine Nüchternplasmaglucosekonzentration von mindestens 8 mmol/l hat.

8. Pharmazeutische Kombination zur Verwendung nach einem der vorhergehenden Ansprüche, wobei der zu behandelnde Patient einen Glucoseexkursion von mindestens 2 mmol/l, mindestens 3 mmol/l, mindestens 4 mmol/l oder mindestens 5 mmol/l hat, wobei es sich bei der Glucoseexkursion um die Differenz zwischen der Plasmaglucosekonzentration 2 Stunden nach einer Mahlzeit und der Plasmaglucosekonzentration 30 Minuten vor einer Testmahlzeit handelt.

9. Pharmazeutische Kombination zur Verwendung nach einem der vorhergehenden Ansprüche, wobei der zu behandelnde Patient orientalischer und/oder asiatischer Rasse ist.

10. Pharmazeutische Kombination zur Verwendung nach einem der vorhergehenden Ansprüche, wobei das desPro³⁶Exendin-4-(1-39)-Lys₆-NH₂ und/oder das pharmazeutisch unbedenkliche Salz davon für die parenterale Verabreichung zubereitet ist.

11. Pharmazeutische Kombination zur Verwendung nach einem der vorhergehenden Ansprüche, wobei das desPro³⁶Exendin-4-(1-39)-Lys₆-NH₂ und/oder das pharmazeutisch unbedenkliche Salz davon für die Verabreichung in einer Tagesdosis ausgewählt aus dem Bereich von 10 µg bis 20 µg zubereitet ist.

12. Pharmazeutische Kombination zur Verwendung nach einem der vorhergehenden Ansprüche, wobei das Metformin und/oder das pharmazeutisch unbedenkliche Salz davon für die orale Verabreichung zubereitet ist.

13. Pharmazeutische Kombination zur Verwendung nach einem der vorhergehenden Ansprüche, wobei der Sulfonylharnstoff für die orale Verabreichung zubereitet ist.

## Revendications

1. Combinaison pharmaceutique pour utilisation dans la diminution de la concentration plasmatique en glucagon chez des patients atteints de diabète de type 2, ladite combinaison comprenant
(a) de la desPro³⁶Exendine-4(1-39)-Lys₆-NH₂ et/ou l'un des sels pharmaceutiquement acceptables de celle-ci,
(b) de la metformine et/ou l'un des sels pharmaceutiquement acceptables de celle-ci, et
(c) une sulfonylurée,
où le sujet à traiter présente une glycémie plasmatique postprandiale à 2 heures d'au moins 14 mmol/L.

2. Combinaison pharmaceutique pour utilisation selon la revendication 1, où le sujet à traiter est obèse.

3. Combinaison pharmaceutique pour utilisation selon l'une quelconque des revendications précédentes, où le sujet à traiter présente un indice de masse corporelle d'au moins 30 kg/m².

4. Combinaison pharmaceutique pour utilisation selon l'une quelconque des revendications précédentes, où le sujet à traiter est un sujet adulte.

5. Combinaison pharmaceutique pour utilisation selon l'une quelconque des revendications précédentes, où chez le sujet à traiter, un diabète sucré de type 2 a été diagnostiqué au moins 1 an ou au moins 2 ans avant le démarrage du traitement.

6. Combinaison pharmaceutique pour utilisation selon l'une quelconque des revendications précédentes, où le sujet à traiter présente une valeur de HbA_{1c} comprise entre environ 7 et environ 10 %.

7. Combinaison pharmaceutique pour utilisation selon l'une quelconque des revendications précédentes, où le sujet à traiter présente une glycémie plasmatique à jeun d'au moins 8 mmol/L.

8. Combinaison pharmaceutique pour utilisation selon l'une quelconque des revendications précédentes, où le sujet à traiter présente une excursion glycémique d'au moins 2 mmol/L, d'au moins 3 mmol/L, d'au moins 4 mmol/L ou d'au moins 5 mmol/L, l'excursion glycémique étant la différence entre la glycémie plasmatique postprandiale à 2 heures et la glycémie plasmatique 30 minutes avant un test de repas.

9. Combinaison pharmaceutique pour utilisation selon l'une quelconque des revendications précédentes, où le sujet à traiter est d'ethnie orientale et/ou asiatique.

10. Combinaison pharmaceutique pour utilisation selon l'une quelconque des revendications précédentes, où la desPro³⁶Exendine-4(1-39)-Lys₆-NH₂ et/ou l'un des sels pharmaceutiquement acceptables de celle-ci sont préparés pour une administration parentérale.

11. Combinaison pharmaceutique pour utilisation selon l'une quelconque des revendications précédentes, où la desPro³⁶Exendine-4(1-39)-Lys₆-NH₂ et/ou l'un des sels pharmaceutiquement acceptables de celle-ci sont préparés pour une administration à une dose journalière choisie dans l'intervalle s'étendant de 10 µg à 20 µg.

12. Combinaison pharmaceutique pour utilisation selon l'une quelconque des revendications précédentes, où la metformine et/ou l'un des sels pharmaceutiquement acceptables de celle-ci sont préparés pour une administration orale.

13. Combinaison pharmaceutique pour utilisation selon l'une quelconque des revendications précédentes, où la sulfonylurée est préparée pour une administration orale.


### SEQUENCE LISTING

<110> Sanofi-Aventis Deutschland GmbH
<120> Pharmaceutical combination for use in glycemic control in diabetes type 2 patients
<130> 50547PEP-1
<140> EP12163637.7
   <141> 2012-04-10
<160> 2
<170> PatentIn version 3.5
<210> 1
   <211> 44
   <212> PRT
   <213> Artificial Sequence
<220>
   <223> AVE0010
<220>
   <221> MOD_RES
   <222> (44)..(44)
   <223> AMIDATION
<400> 1
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Ser Lys Lys Lys Lys Lys Lys
           35                  40
<210> 2
   <211> 39
   <212> PRT
   <213> Artificial Sequence
<220>
   <223> Exendin-4
<220>
   <221> MOD_RES
   <222> (39)..(39)
   <223> AMIDATION
<400> 2
   His Gly Glu Gly Thr Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
                   20                  25                  30
   Ser Gly Ala Pro Pro Pro Ser
           35